(19) Europäisches Patentamt
European Patent Office
Office européen des brevets

(11) EP 1 518 535 B1

(12) **FASCICULE DE BREVET EUROPEEN**

(45) Date de publication et mention de la délivrance du brevet:
**12.08.2009 Bulletin 2009/33**

(51) Int Cl.:
***A61K 8/81*** *(2006.01)* ***A61K 8/89*** *(2006.01)*
***A61Q 1/00*** *(2006.01)* ***A61Q 19/00*** *(2006.01)*

(21) Numéro de dépôt: **04292212.0**

(22) Date de dépôt: **15.09.2004**

(54) **Composition cosmétique comprenant un polymère séquencé et une huile siliconée non volatile**

Kosmetische Zusammensetzung enthaltend einen Blockpolymer und ein nicht flüchtiges Silikonöl

Cosmetic composition comprising a blockpolymer and a non volatile silicone oil

(84) Etats contractants désignés:
**AT BE BG CH CY CZ DE DK EE ES FI FR GB GR HU IE IT LI LU MC NL PL PT RO SE SI SK TR**

(30) Priorité: **26.09.2003 FR 0311337**

(43) Date de publication de la demande:
**30.03.2005 Bulletin 2005/13**

(73) Titulaire: **L'ORÉAL**
**75008 Paris (FR)**

(72) Inventeurs:
- **Blin, Xavier**
**75015 Paris (FR)**
- **Ferrari, Véronique**
**94700 Maisons-Alfort (FR)**

(74) Mandataire: **Kromer, Christophe**
**L'OREAL - D.I.P.I.**
**25-29 Quai Aulagnier**
**92600 Asnières (FR)**

(56) Documents cités:
**EP-A- 1 366 746**
**WO-A-02/067877**
**WO-A-03/046032**
**FR-A- 2 832 720**
**US-A- 5 690 918**
**US-A1- 2002 115 780**

Remarques:
Le dossier contient des informations techniques présentées postérieurement au dépôt de la demande et ne figurant pas dans le présent fascicule.

EP 1 518 535 B1

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

## Description

La présente invention a pour objet une composition cosmétique comprenant un polymère séquencé particulier et une huile siliconée non volatile destinée à être appliquée sur les matières kératiniques d'êtres humains, comme la peau, les lèvres, les cils, les sourcils, les ongles, les cheveux. La composition est plus particulièrement destinée à être appliquée sur la peau ou les lèvres.

La composition selon l'invention peut être une composition de maquillage ou une composition de soin des matières kératiniques, en particulier de la peau et des lèvres, et de préférence une composition de maquillage.

La composition de maquillage peut être un produit de maquillage des lèvres (rouge à lèvres), un fond de teint, un fard à paupières, un fard à joue, un produit anticernes, un eye-liner, un produit de maquillage du corps, un mascara, un vernis à ongles, un produit de maquillage des cheveux.

La composition de soin peut être un produit de soin de la peau du corps et du visage, notamment un produit solaire, un produit de coloration de la peau (tel qu'un autobronzant). La composition peut être également un produit capillaire, notamment pour le maintien de la coiffure ou la mise en forme des cheveux.

Les compositions de rouge à lèvres et fond de teint sont couramment employées pour apporter une couleur esthétique aux lèvres ou à la peau, notamment au visage. Ces produits de maquillage contiennent généralement des phases grasses telles que des cires et des huiles, des pigments et/ou charges et éventuellement des additifs comme des actifs cosmétiques ou dermatologiques.

Ces compositions, lorsqu'elles sont appliquées sur la peau, présentent l'inconvénient de transférer, c'est-à-dire de se déposer au moins en partie, en laissant des traces, sur certains supports avec lesquels elles peuvent être mises en contact et notamment un verre, une tasse, une cigarette, un vêtement ou la peau. Il s'ensuit une persistance médiocre du film appliqué, nécessitant de renouveler régulièrement l'application de la composition de fond de teint ou de rouge à lèvres. Par ailleurs, l'apparition de ces traces inacceptables notamment sur les cols de chemisier peut écarter certaines femmes de l'utilisation de ce type de maquillage.

On recherche donc des compositions de maquillage pour les lèvres et la peau dites « sans transfert » qui présentent l'avantage de former un dépôt qui ne se dépose pas, au moins en partie, sur les supports avec lesquels elles sont mises en contact (verre, vêtements, cigarette, tissus).

Les compositions sans transfert connues sont généralement à base de résines de silicone et d'huiles de silicone volatiles mais ces compositions ont l'inconvénient de laisser sur la peau et les lèvres, après évaporation des huiles de silicone volatiles, un dépôt conférant à l'utilisatrice une sensation de dessèchement et de tiraillement : le dépôt de maquillage dévient donc inconfortable au cours du temps. De plus, certaines résines de silicone peuvent former un dépôt de maquillage collant et donc rendre le maquillage encore plus inconfortable.

Pour diminuer l'effet inconfortable du maquillage, il est possible d'ajouter des huiles non volatiles hydrocarbonées comme le polyisobutylène mais on a alors constaté qu'en employant uniquement des huiles non volatiles hydrocarbonées, la propriété non transfert du maquillage est altérée.

La présente invention a donc pour but de fournir une composition cosmétique présentant de bonnes propriétés de non transfert et formant un dépôt sur les matières kératiniques, en particulier sur la peau ou les lèvres, confortable au cours du temps.

Les inventeurs ont découvert qu'il est possible d'obtenir une telle composition en utilisant un polymère séquencé particulier associé à une huile siliconée non volatile présente en une quantité suffisante.

De façon plus précise, la présente invention a donc pour objet une composition cosmétique comprenant un polymère séquencé et un milieu liquide organique cosmétiquement acceptable contenant une phase grasse liquide non volatile, caractérisée par le fait que :

- le polymère séquencé est un polymère éthylénique linéaire filmogène.- et que la phase grasse liquide non volatile comprend au moins 30 % en poids, par rapport au poids total de la phase grasse liquide non volatile, d'huile siliconée non volatile.

Avantageusement, le polymère séquencé est exempt de styrène.

Avantageusement encore, le polymère séquencé est non élastomère.

De préférence encore, le polymère séquencé comprend moins une première séquence et au moins une deuxième séquence ayant des températures de transition vitreuse (Tg) différentes, lesdites première et deuxième séquences étant reliées entre elles par une séquence intermédiaire comprenant au moins un monomère constitutif de la première séquence et au moins un monomère constitutif de la deuxième séquence.

De préférence, le polymère a un indice de polydispersité I supérieur à 2,

De préférence également, les première et seconde séquences sont incompatibles l'une avec l'autre.

La composition selon l'invention permet d'obtenir un dépôt, notamment un dépôt de maquillage, sur les matières kératiques, en particulier sur la peau ou les lèvres, présentant de bonnes propriétés de sans transfert, sans sensation

de dessèchement, de tiraillement ou de collant : le dépôt ainsi obtenu est donc confortable au cours du temps pour l'utilisatrice.

L'invention a également pour objet un procédé de maquillage des matières kératiniques, en particulier de la peau ou des lèvres, comprenant l'application sur les matières kératiniques, en particulier sur la peau ou les lèvres, d'une composition telle que définie précédemment.

L'invention a aussi pour objet l'utilisation d'une composition telle que définie précédemment pour obtenir un dépôt sur les matières kératiniques, en particulier sur la peau ou les lèvres, ayant des propriétés de sans transfert et confortable au cours du temps.

L'invention a encore pour objet l'utilisation, dans une composition cosmétique comprenant un milieu liquide organique cosmétiquement acceptable contenant une phase grasse liquide non volatile,

- d'un polymère éthylénique séquencé, linéaire filmogène,
- et d'une huile siliconée non volatile présente en une teneur d'au moins 30 % en poids, par rapport au poids total de la phase grasse liquide non volatile,

pour obtenir un dépôt sur les matières kératiniques, en particulier sur la peau ou les lèvres, ayant des propriétés de sans transfert et confortable au cours du temps.

Par milieu liquide organique cosmétiquement acceptable, on entend un milieu comprenant au moins un composé organique liquide à température ambiante (25 °C) et pression atmosphérique ($10^5$ Pa) compatible avec les matières kératiniques, notamment la peau, les lèvres, telles que les huiles ou les solvants organiques couramment employés dans les compositions cosmétiques.

Le polymère séquencé de la composition selon l'invention est un polymère éthylénique séquencé linéaire filmogène.

Par polymère "éthylénique" , on entend un polymère obtenu par polymérisation de monomères comprenant une insaturation éthylénique.

Par polymère "séquencé", on entend un polymère comprenant au moins 2 séquences distinctes, de préférence au moins 3 séquences distinctes.

Le polymère est un polymère à structure linéaire. Par opposition, un polymère a structure non linéaire est, par exemple, un polymère à structure ramifiée, en étoile, greffée, ou autre.

Par polymère "filmogène", on entend un polymère apte à former à lui seul ou en présence d'un agent auxiliaire de filmification, un film continu et adhérent sur un support, notamment sur les matières kératiniques.

La composition selon l'invention comprend avantageusement un polymère séquencé comprenant au moins une première séquence et au moins une deuxième séquence, de préférence incompatibles l'une avec l'autre, et ayant des températures de transition vitreuse (Tg) différentes, lesdites première et deuxième séquences étant reliées entre elles par une séquence intermédiaire comprenant au moins un monomère constitutif de la première séquence et au moins un monomère constitutif de la deuxième séquence.

On précise que dans ce qui précède et ce qui suit les termes "première" et "deuxième" séquences ne conditionnent nullement l'ordre desdites séquences (ou blocs) dans la structure du polymère.

Par "au moins" une séquence, on entend une ou plusieurs séquences.

Par "séquences incompatibles l'une avec l'autre", on entend que le mélange formé du polymère correspondant à la première séquence et du polymère correspondant à la deuxième séquence, n'est pas miscible dans le liquide organique majoritaire en poids du milieu liquide organique de la composition, à température ambiante (25°C) et pression atmosphérique ($10^5$ Pa), pour une teneur du mélange de polymères supérieure ou égale à 5 % en poids, par rapport au poids total du mélange (polymères et solvant), étant entendu que :

i) lesdits polymères sont présents dans le mélange en une teneur telle que le rapport pondéral respectif va de 10/90 à 90/10, et que

ii) chacun des polymères correspondant au première et seconde séquences a une masse moléculaire moyenne (en poids ou en nombre) égale à celle du polymère séquencé +/- 15%.

Dans le cas où le milieu liquide organique comprend un mélange de liquide organiques, et dans l'hypothèse de deux ou plusieurs liquides organiques présents en proportions massiques identiques, ledit mélange de polymères est non miscible dans au moins l'un d'entre eux.

Bien entendu, dans le cas où le milieu liquide organique comprend un unique liquide organique, ce dernier est le liquide organique majoritaire.

Avantageusement, le liquide organique majoritaire de la composition est le solvant organique de polymérisation du polymère séquencé ou le solvant organique majoritaire du mélanges de solvant organiques de polymérisation du polymère séquencé.

La séquence intermédiaire est une séquence comprenant au moins un monomère constitutif de la première séquence et au moins un monomère constitutif de la deuxième séquence du polymère permet de "compatibiliser" ces séquences.

De façon préférentielle, le polymère utilisé dans la composition selon l'invention ne comprend pas d'atomes de silicium dans son squelette. Par "squelette", on entend la chaîne principale du polymère, par opposition aux chaînes latérales pendantes.

De préférence, le polymère utilisé dans la composition selon l'invention n'est pas hydrosoluble, c'est à dire que le polymère n'est pas soluble dans l'eau ou dans un mélange d'eau et de monoalcools inférieurs linéaires ou ramifiés ayant de 2 à 5 atomes de carbone comme l'éthanol, l'isopropanol ou le n-propanol, sans modification de pH, à une teneur en matière active d'au moins 1% en poids, à température ambiante (25°C).

Avantageusement, le polymère séquencé utilisé selon l'invention est présent dans le milieu liquide organique de la composition.

De préférence, le polymère utilisé dans la composition selon l'invention n'est pas un élastomère.
Par "polymère non élastomère", on entend un polymère qui, lorsqu'il est soumis à une contrainte visant à l'étirer (par exemple de 30% relativement à sa longueur initiale), ne revient pas à une longueur sensiblement identique à sa longueur initiale lorsque cesse la contrainte.

De manière plus spécifique, par "polymère non élastomére" on désigne un polymère ayant une recouvrance instantanée $R_i$ < à 50% et une recouvrance retardée $R_{2h}$ < 70% après avoir subi un allongement de 30%. De préférence, $R_i$ est < à 30 %, et $R_{2h}$ < 50 %.

Plus précisément, le caractère non élastomérique du polymère est déterminé selon le protocole suivant :

On prépare un film de polymère par coulage d'une solution du polymère dans une matrice téflonnée puis séchage pendant 7 jours dans une ambiance contrôlée à 23±5°C et 50±10 % d'humidité relative.
On obtient alors un film d'environ 100 µm d'épaisseur dans lequel sont découpées des éprouvettes rectangulaires (par exemple à l'emporte-pièce) d'une largeur de 15 mm et d'une longueur de 80 mm.

On impose à cet échantillon une sollicitation de traction à l'aide d'un appareil commercialisé sous la référence Zwick, dans les mêmes conditions de température et d'humidité que pour le séchage.
Les éprouvettes sont étirées à une vitesse de 50 mm/min et la distance entre les mors est de 50 mm, ce qui correspond à la longueur initiale ($l_0$) de l'éprouvette.

On détermine la recouvrance instantanée Ri de la manière suivante :

- on étire l'éprouvette de 30 % ($\varepsilon_{max}$) c'est-à-dire environ 0,3 fois sa longueur initiale ($l_0$)
- on relâche la contrainte en imposant une vitesse de retour égale à la vitesse de traction, soit 50 mm/min et on mesure l'allongement résiduel de l'éprouvette en pourcentage, après retour à contrainte nulle ($\varepsilon_i$).

La recouvrance instantanée en % ($R_i$) est donnée par la formule ci-après:

$$R_i = (\varepsilon_{max} - \varepsilon_i)/\ \varepsilon_{max}) \times 100$$

Pour déterminer la recouvrance retardée, on mesure l'allongement résiduel de l'éprouvette en pourcentage ($\varepsilon_{2h}$).

La recouvrance retardée en % ($R_{2h}$) est donnée par la formule ci-après:

$$R_{2h} = (\varepsilon_{max} - \varepsilon_{2h})/\ \varepsilon_{max}) \times 100$$

A titre purement indicatif, un polymère selon un mode de réalisation de l'invention possède une recouvrance instantanée $R_i$ de 10% et une recouvrance retardée $R_{2h}$ de 30%.

De préférence, le polymère séquencé utilisé dans les compositions selon l'invention a un indice de polydispersité I supérieur à 2.

L'indice de polydispersité I du polymère est égal au rapport de la masse moyenne en poids Mw sur la masse moyenne en nombre Mn.

On détermine les masses molaires moyennes en poids (Mw) et en nombre (Mn) par chromatographie liquide par perméation de gel (solvant THF, courbe d'étalonnage établie avec des étalons de polystyrène linéaire, détecteur

réfractométrique).

La masse moyenne en poids (Mw) du polymère utilisé dans la composition selon l'invention est de préférence inférieure ou égale à 300 000, elle va par exemple de 35 000 à 200 000, et mieux de 45 000 à 150 000.

La masse moyenne en nombre (Mn) du polymère utilisé dans la composition selon l'invention est de préférence inférieure ou égale à 70 000, elle va par exemple de 10 000 à 60 000, et mieux de 12 000 à 50 000.

L'indice de polydispersité du polymère utilisé dans la composition selon l'invention est supérieur à 2, par exemple est supérieur à 2 et inférieur ou égal à 9, de préférence supérieur ou égal à 2,5, par exemple allant de 2,5 à 8, et mieux supérieur ou égal à 2,8 , notamment de 2,8 à 6.

Chaque séquence ou bloc du polymère utilisé dans la composition selon l'invention est issue d'un type de monomère ou de plusieurs types de monomères différents.

Cela signifie que chaque séquence peut être constituée d'un homopolymère ou d'un copolymère ; ce copolymère constituant la séquence pouvant être à son tour statistique ou alterné.

Avantageusement, la séquence intermédiaire comprenant au moins un monomère constitutif de la première séquence et au moins un monomère constitutif de la deuxième séquence du polymère est un polymère statistique.

De préférence, la séquence intermédiaire est issue essentiellement de monomères constitutifs de la première séquence et de la deuxième séquence.

Par "essentiellement", on entend au moins à 85%, de préférence au moins à 90%, mieux à 95% et encore mieux à 100%.

Avantageusement, la séquence intermédiaire a une température de transition vitreuse Tg comprise entre les températures de transition vitreuse des première et deuxième séquences.

Les températures de transition vitreuse indiquées des première et deuxième séquences peuvent être des Tg théoriques déterminées à partir des Tg théoriques des monomères constitutifs de chacune des séquences, que l'on peut trouver dans un manuel de référence tel que le Polymer Handbook, 3rd ed, 1989, John Wiley, selon la relation suivante, dite Loi de Fox :

$$1/Tg = \sum_i (\varpi_i / Tg_i) ,$$

$\omega_i$ étant la fraction massique du monomère i dans la séquence considerée et $Tg_i$ étant la température de transition vitreuse de l'homopolymère du monomère i.

Sauf indication contraire, les Tg indiquées pour les première et deuxième séquences dans la présente demande sont des Tg théoriques.

La première séquence est une séquence ayant une Tg supérieure ou égale à 40°C, et la deuxième séquence est une séquence ayant une Tg inférieure ou égale à 20°C.

a) Séquence ayant une Tg supérieure ou égale à 40°C

La séquence ayant une Tg supérieure ou égale à 40°C a par exemple une Tg allant de 40 à 150°C, de préférence supérieure ou égale à 50°C, allant par exemple de 50°C à 120 °C, et mieux supérieure ou égale à 60°C, allant par exemple de 60°C à 120°C .

La séquence ayant une Tg supérieure ou égale à 40°C est issue de monomère dont l'homopolymère a une température de transition vitreuse supérieure ou égale à 40°C est issue de monomère dont l'homopolymère a une température de transition vitreuse supérieure ou égale à 40°C. .

Les monomères dont l'homopolymère a une température de transition vitreuse supérieure ou égale à 40°C choisis parmi les monomères suivants, appelés aussi monomères principaux :

- le méthacrylate d'isobornyle
- l'acrylate d'isobornyle
- et leurs mélanges.

b) Séquence ayant une Tg inférieure ou égale à 20°C

La séquence ayant une Tg inférieure ou égale à 20°C a par exemple une Tg allant de -100 à 20°C, de préférence inférieure ou égale à 15°C, notamment allant de -80°C à 15°C et mieux inférieure ou égale à 10°C, par exemple allant de -50°C à 0°C.

La séquence ayant une Tg inférieure ou égale à 20°C est un homopolymère, issue de monomères, qui sont tel(s) que les homopolymères préparés à partir de ces monomères ont des températures de transition vitreuse inférieures ou égales à 20°C. Cette deuxième séquence peut être un homopolymère, constitué par un seul type de monomère (dont la Tg de l'homopolymère correspondant est inférieure ou égale à 20°C.

Les monomères dont l'homopolymère a une Tg inférieure ou égale à 20°C sont choisis parmi les monomères suivants, ou monomère principaux :

- les acrylates de formule $CH_2 = CHCOOR_3$,
  $R_3$ représentant un groupe alkyle non substitué en $C_1$ à $C_{12}$, linéaire ou ramifié, à l'exception du groupe tertiobutyle, dans lequel se trouve(nt) éventuellement intercalé(s) un ou plusieurs hétéroatomes choisis parmi O, N, S.

Les monomères principaux particulièrement préférés pour la séquence ayant une Tg inférieure ou égale à 20°C sont les acrylates d'alkyles dont la chaîne alkyle comprend de 1 à 10 atomes de carbone, à l'exception du groupe tertiobutyle, tels que l'acrylate de méthyle, l'acrylate d'isobutyle, l'acrylate d'éthyl-2 hexyle et leurs mélanges.

De préférence, la proportion de la deuxième séquence ayant une Tg inférieure ou égale à 20°C va de 10 à 85% en poids du polymère, mieux de 20 à 70% et encore mieux de 20 à 50%.

Selon un mode de réalisation, le polymère séquencé utilisé dans la composition selon l'invention est exempt de styrène. Par polymère exempt de styrène, on entend un polymère comprenant moins de 10%, de préférence moins de 5%, de préférence moins de 2%, de préférence encore moins de 1% en poids, voire ne contient pas, de monomère styrénique tels que le styrène ou les dérivés du styrène comme par exemple le méthylstyrène, le chlorostyrène ou le chlorométhylstyrène.

Selon un mode de réalisation, le polymère séquencé de la composition selon l'invention est issu de monomères éthyléniques aliphatiques. Par monomère aliphatique, on entend un monomère ne comprenant aucun groupe aromatique.

Chacune des séquences peut néanmoins contenir en proportion minoritaire au moins un monomère constitutif de l'autre séquence.
Ainsi la première séquence peut contenir au moins un monomère constitutif de la deuxième séquence et inversement.

Chacune des première et/ou deuxième séquence, peu(ven)t comprendre, outre les monomères indiqués ci-dessus, un ou plusieurs autres monomères appelés monomères additionnels, différents des monomères principaux cités précédemment.
La nature et la quantité de ce ou ces monomères additionnels sont choisies de manière à ce que la séquence dans laquelle ils se trouvent ait la température de transition vitreuse désirée.

Ce monomère additionnel est par exemple choisi parmi :

les monomères hydrophiles tels que :

- les monomères à insaturation(s) éthylénique(s) comprenant au moins une fonction acide carboxylique ou sulfonique comme par exemple :
  l'acide acrylique, l'acide méthacrylique, l'acide crotonique, l'anhydride maléique, l'acide itaconique, l'acide fumarique, l'acide maléique, l'acide acrylamidopropanesulfonique, l'acide vinylbenzoïque, l'acide vinylphosphorique et les sels de ceux-ci,
- les monomères à insaturation(s) éthylénique(s) comprenant au moins une fonction amine tertiaire comme la 2-vinylpyridine, la 4-vinylpyridine, le méthacrylate de diméthylaminoéthyle, le méthacrylate de diéthylaminoéthyle, le diméthylaminopropyl méthacrylamide et les sels de ceux-ci,
- les méthacrylates de formule $CH_2 = C(CH_3)\text{-}COOR_6$
  dans laquelle $R_6$ représente un groupe alkyle linéaire ou ramifié, contenant de 1 à 4 atomes de carbone, tel qu'un groupe méthyle, éthyle, propyle ou isobutyle, ledit groupe alkyle étant substitué par un ou plusieurs substituants choisis parmi les groupes hydroxyle (comme le méthacrylate de 2-hydroxypropyle, le méthacrylate de 2-hydroxyéthyle) et les atomes d'halogènes (Cl, Br, I, F), tel que le méthacrylate de trifluoroéthyle,
- les méthacrylates de formule $CH_2 = C(CH_3)\text{-}COOR_9$,
  $R_9$ représentant un groupe alkyle en $C_6$ à $C_{12}$ linéaire ou ramifié, dans lequel se trouve(nt) éventuellement intercalé(s) un ou plusieurs hétéroatomes choisis parmi 0, N et S, ledit groupe alkyle étant substitué par un ou plusieurs substituants choisis parmi les groupes hydroxyle et les atomes d'halogènes (Cl, Br, I, F) ;
- les acrylates de formule $CH_2 = CHCOOR_{10}$,
  $R_{10}$ représentant un groupe alkyle en $C_1$ à $C_{12}$ linéaire ou ramifié substitué par un ou plusieurs substituants choisis parmi les groupes hydroxyle et les atomes d'halogène (Cl, Br, I et F), tel que l'acrylate de 2-hydroxypropyle et l'acrylate de 2-hydroxyéthyle, ou $R_{10}$ représente un alkyle($C_1$-$C_{12}$)-O-POE (polyoxyéthylène) avec répétition du motif oxyéthylène de 5 à 30 fois, par exemple méthoxy-POE, ou $R_{10}$ représente un groupement polyoxyé-

thylèné comprenant de 5 à 30 motifs d'oxyde d'éthylène

b) les monomères à insaturation éthylénique comprenant un ou plusieurs atomes de silicium tels que le méthacryloxypropyl triméthoxy silane, le méthacryloxypropyl tris ( triméthylsiloxy ) silane,

- et leurs mélanges.

Des monomères additionnels particulièrement préférés sont l'acide acrylique, l'acide méthacrylique, le méthacrylate de trifluoroéthyle et leurs mélanges.

Ce ou ces monomères additionnels représente(nt) généralement une quantité inférieure ou égale à 30% en poids, par exemple de 1 à 30% en poids, de préférence de 5 à 20% en poids et, de préférence encore, de 7 à 15% en poids du poids total des première et/ou deuxième séquences.

Selon un mode préféré de réalisation, le polymère utilisé dans la composition selon l'invention est un polymère non siliconé, c'est à dire un polymère exempt d'atome de silicium.

De préférence, chacune des première et deuxième séquences comprend au moins un monomère choisi parmi les esters d'acide (méth)acrylique tels que définis précédemment, et éventuellement un monomère choisi parmi l'acide (méth)acrylique, et leurs mélanges.

Avantageusement, chacune des première et deuxième séquences est issue en totalité d'au moins un monomère choisi parmi les esters d'acide (méth)acrylique tels que définis précédemment, et éventuellement un monomère choisi parmi l'acide (méth)acrylique, et leurs mélanges.

Le polymère utilisé dans la composition selon l'invention peut être obtenu par polymérisation radicalaire en solution selon le procédé de préparation suivant :

une partie du solvant de polymérisation est introduite dans un réacteur adapté et chauffée jusqu'à atteindre la température adéquate pour la polymérisation (typiquement entre 60 et 120°C),

une fois cette température atteinte, les monomères constitutifs de la première séquence sont introduits en présence d'une partie de l'initiateur de polymérisation, au bout d'un temps T correspondant à un taux de conversion maximum de 90%, les monomères constitutifs de la deuxième séquence et l'autre partie de l'initiateur sont introduits,

on laisse réagir le mélange pendant un temps T' ( allant de 3 à 6 h) au bout duquel le mélange est ramené à température ambiante,

on obtient le polymère en solution dans le solvant de polymérisation.

Par solvant de polymérisation, on entend un solvant ou un mélange de solvants. Le solvant de polymérisation peut être choisis notamment parmi l'acétate d'éthyle, l'acétate de butyle, les alcools tels que l'isopropanol, l'éthanol, les alcanes aliphatiques tels que l'isododécane et leurs mélanges. De préférence, le solvant de polymérisation est un mélange acétate de butyle et isopropanol ou l'isododécane.

De préférence, la proportion de la séquence ayant une Tg supérieure ou égale à 40°C va de 20 à 90% en poids du polymère, mieux de 30 à 80% et encore mieux de 50 à 70%.

De préférence, la proportion de la séquence ayant une Tg inférieure ou égale à 20°C va de 5 à 75% en poids du polymère, de préférence de 15 à 50% et mieux de 25 à 45%.

Le polymère séquencé décrit précédemment peut être présent dans la composition selon l'invention en une teneur allant de 0,1 % à 90 % en poids, par rapport au poids total de la composition, de préférence allant de 0,5 % à 50 % en poids, et préférentiellement allant de 0,5 % à 30 % en poids.

La composition selon l'invention comprend une huile siliconée.

Par "huile", on entend tout milieu non aqueux liquide à température ambiante (25°C) et pression atmosphérique (760mm de Hg), compatible avec une application sur la peau, les muqueuses (lèvres) et/ou les phanères (ongles, cils, sourcils, cheveux).

On entend par huile non volatile une huile susceptible de rester sur la peau à température ambiante (25 °C) et pression atmosphérique au moins une heure et ayant notamment une pression de vapeur à température ambiante (25 °C) et pression atmosphérique, non nulle, inférieure à 0,01 mm de Hg (1,33 Pa).

L'huile siliconée non volatile a de préférence une viscosité comprise dans la gamme allant de 10 à 10 000 cSt à 25°C et mieux de 10 à 5 000 cSt.

L'huile non volatile siliconée peut être choisie parmi les polydiméthylsiloxanes (PDMS) non volatils ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényl triméthicones, les phényl diméthicones, les phényl triméthylsiloxy diphénylsiloxanes, les diphényl diméthicones, les diphényl méthyldiphényl trisiloxanes ; les polysiloxanes modifiés par des acides gras (notamment en $C_8$-$C_{20}$), des alcools gras (notamment en $C_8$-$C_{20}$) ou des polyoxyalkylènes (notamment polyoxyéthyléne et/ou polyoxypropylène) ; les silicones aminées ; les silicones à groupement hydroxyles ; les silicones fluorés comportant un groupement fluoré pendant ou en bout de chaîne siliconée ayant de 1 à 12 atomes de carbone dont tout ou partie des hydrogène sont substitués par des atomes de fluor ;

et leurs mélanges.

Avantageusement, l'huile siliconée non volatile est choisie parmi les huiles siliconées phénylées non volatiles.

L'huile siliconée phénylée non volatile peut être choisie parmi les silicones phénylées de formule (VI) suivante :

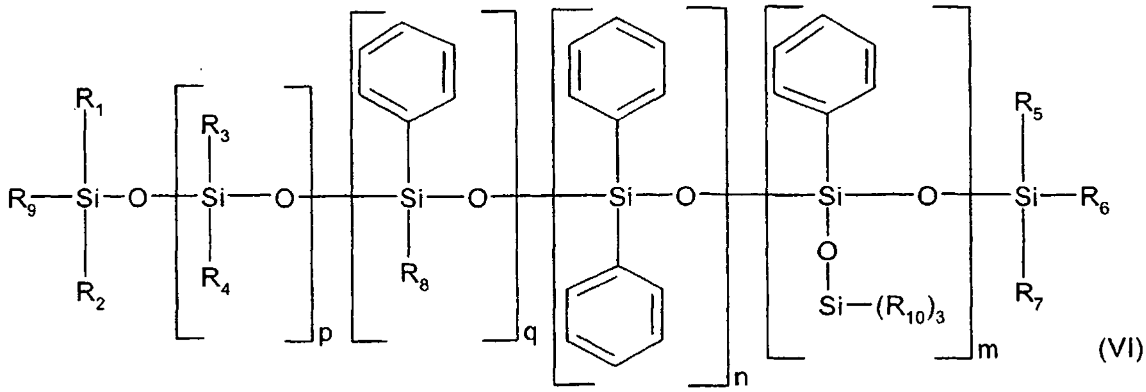

(VI)

dans laquelle

- R1 à R10, indépendamment les uns des autres, sont des radicaux hydrocarbonés, saturés ou insaturés, linéaires, cycliques ou ramifiés, en C1-C30,
- m, n, p et q sont, indépendamment les uns des autres, des nombres entiers compris entre 0 et 900, sous réserve que la somme 'm+n+q' est différente de 0.

De préférence, la somme 'm+n+q' est comprise entre 1 et 100.
De préférence, la somme 'm+n+p+q' est comprise entre 1 et 900, encore mieux entre 1 et 800.
De préférence, q est égal à 0.

De préférence, l'huile siliconée phénylée non volatile est choisie parmi les silicones phénylées de formule (VII) suivante :

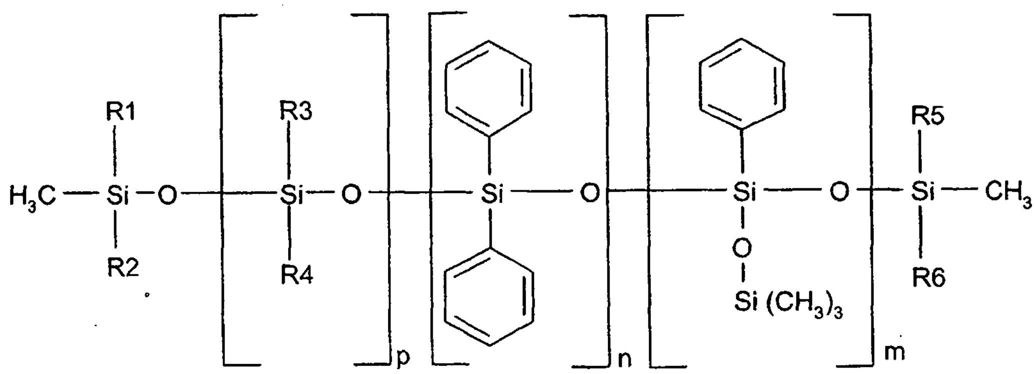

(VII)

dans laquelle :

- R1 à R6, indépendamment les uns des autres, sont des radicaux hydrocarbonés, saturés ou insaturés, linéaires cycliques ou ramifiés, en C1-C30,
- m, n et p sont, indépendamment les uns des autres, des nombres entiers compris entre 0 et 100, sous réserve que la somme 'n + m' est comprise entre 1 et 100.

De préférence, R1 à R6, indépendamment les uns des autres, représentent un radical hydrocarboné saturé, linéaire ou ramifié, en C1-C30, notamment en C1-C12, et en particulier un radical méthyle, éthyle, propyle ou butyle.

Notamment, R1 à R6 peuvent être identiques, et en outre peuvent être un radical méthyle.

De préférence, on peut avoir m=1 ou 2 ou 3, et/ou n=0 et/ou p=0 ou 1.

Les huiles siliconées phénylées peuvent être choisies parmi les phényl trimethicones, les phényl dimethicones, les phényl triméthylsiloxy diphénylsiloxanes, les diphényl diméthicones, les diphényl méthyldiphényl trisiloxanes, et leurs mélanges.

De préférence, le poids moléculaire en poids de l'huile siliconée phénylée est compris entre 500 et 10 000.

Avantageusement, on utilise une huile siliconée phénylée de formule (VI) ayant une viscosité à 25°C comprise entre 5 et 1500 $mm^2/s$ (soit 5 à 1500 cSt), de préférence ayant une viscosité comprise entre 5 et 1000 $mm^2/s$ (soit 5 à 1000 cSt).

Comme huile sliconée phénylée non volatile, on peut utiliser notamment les phényltriméthicones telles que la DC556 de Dow Corning (22,5 cSt), l'huile Silbione 70663V30 de Rhône Poulenc (28 cSt), ou les diphényldiméthicones telles que les huiles Belsil, notamment Belsil PDM1000 (1000cSt), Belsil PDM 200 (200 cSt) et Belsil PDM 20 (20cSt) de Wacker. Les valeurs entre parenthèses représentent les viscosités à 25°C.

L'huile siliconée non volatile peut être présente dans la composition selon l'invention en une teneur allant de 30 % à 95 % en poids, par rapport au poids total de la phase grasse liquide non volatile, de préférence allant de 40 % à 85 % en poids, et préférentiellement allant de 50 % à 80 % en poids.

L'huile siliconée non volatile peut être présente dans la composition selon l'invention en une teneur allant de 0,1 % à 70 % en poids, par rapport au poids total de la composition, de préférence allant de 1 % à 50 % en poids, et préférentiellement allant de 1 % à 30 % en poids.

Selon un mode de réalisation particulièrement préféré, le milieu liquide organique de la composition contient au moins un liquide organique qui est le ou un des solvants organique(s) de polymérisation du polymère séquencé tel que décrit précédemment. Avantageusement, ledit solvant organique de polymérisation est le liquide organique majoritaire en poids dans le milieu liquide organique de la composition cosmétique.

La phase grasse liquide non volatile de la composition selon l'invention peut comprendre en outre au moins une huile non volatile non siliconée, notamment une huile non volatile hydrocarbonée. De préférence, la phase grasse liquide non volatile huileuse est macroscopiquement homogène, c'est-à-dire homogène à l'oeil nu.

Par huile hydrocarbonée, on entend une huile formée essentiellement, voire constituée, d'atomes de carbone et d'hydrogène, et éventuellement d'atomes d'oxygène, d'azote, et ne contenant pas d'atome de silicium ou de fluor. Elle peut contenir des groupes alcool, ester, éther, acide carboxylique, amine et/ou amide.

L'huile non volatile non siliconée peut être présente en une teneur allant de 0,1 % à 70 % en poids, par rapport au poids total de la phase grasse liquide non volatile, de préférence allant de 0,5 % à 60 % en poids, et préférentiellement allant de 1 % à 50 % en poids.

L'huile non volatile non siliconée peut être présente dans la composition selon l'invention en une teneur allant de 0,1 % à 60 % en poids, par rapport au poids total de la composition, de préférence allant de 0,5 % à 30 % en poids, et préférentiellement allant de 1 % à 20 % en poids.

Comme huile non volatile non siliconée, on peut utiliser les huiles non volatile hydrocarbonées telles que l'huile de paraffine ( ou vaseline), le squalane, le polyisobutylène hydrogéné (huile de Parléam), le perhydrosqualène, l'huile de vison, de tortue, de soja, l'huile d'amande douce, de calophyllum, de palme, de pépins de raisin, de sésame, de maïs, d'arara, de colza, de tournesol, de coton, d'abricot, de ricin, d'avocat, de jojoba, d'olive ou de germes de céréales ; des esters d'acide lanolique, d'acide oléique, d'acide laurique, d'acide stéarique ; les esters gras, notamment en $C_{12}$-$C_{36}$, tels que le myristate d'isopropyle, le palmitate d'isopropyle, le stéarate de butyle, le laurate d'hexyle, l'adipate de diisopropyle, l'isononanoate d'isononyle, le palmitate de 2-éthyl-hexyle, le laurate de 2-hexyl-décyle, le palmitate de 2-octyldécyle, le myristate ou le lactate de 2-octyl-dodécyle, le succinate de di(2-éthyl hexyle), le malate de diisostéaryle, le triisostéarate de glycérine ou de diglycérine ; les acides gras supérieurs, notamment en $C_{14}$-$C_{22}$, tels que l'acide myristique, l'acide palmitique, l'acide stéarique, l'acide béhénique, l'acide oléique, l'acide linoléique, l'acide linolénique ou l'acide isostéarique ; les alcools gras supérieurs, notamment en $C_{16}$- $C_{22}$, tels que le cétanol, l'alcool oléique, l'alcool linoléique ou linolénique, l'alcool isostéarique ou l'octyl dodécanol ; et leurs mélanges.

Avantageusement, l'huile non volatile non siliconée est choisie parmi les hydrocarbures notamment les alcanes comme le polyisobutène hydrogéné.

La composition selon l'invention peut comprendre en outre au moins une huile volatile.

Par huile volatile, on entend tout milieu non aqueux susceptible de s'évaporer de la peau ou des lèvres, en moins d'une heure, ayant notamment une pression de vapeur, à température ambiante et pression atmosphérique allant de $10^{-3}$ à 300 mm de Hg (0,13 Pa à 40 000 Pa).

Selon l'invention, on peut utiliser une ou plusieurs huiles volatiles.

Ces huiles peuvent être des huiles hydrocarbonées ou des huiles siliconées comportant éventuellement des groupements alkyle ou alkoxy pendants ou en bout de chaîne siliconée.

Comme huile de silicone volatile utilisable dans l'invention, on peut citer les silicones linéaires ou cycliques ayant de 2 à 7 atomes de silicium, ces silicones comportant éventuellement des groupes alkyle ou alkoxy ayant de 1 à 10 atomes de carbone. Comme huile de silicone volatile utilisable dans l'invention, on peut citer notamment l'octaméthylcyclotétrasiloxane, le décaméthylcyclopentasiloxane, le dodécaméthylcyclohexasiloxane, l'heptaméthylhexyltri-siloxane, l'heptaméthyloctyltrisiloxane, l'octaméthyltrisiloxane, le décaméthyltétrasiloxane et leurs mélanges.

Comme autre huile volatile utilisable dans l'invention, on préfère notamment les isoparaffines en $C_8$-$C_{16}$ comme l'isododécane, l'isodécane, l'isohexadécane et par exemple les huiles vendus sous les noms commerciaux d'ISOPAR, de PERMETHYL et notamment l'isododécane (PERMETHYL 99 A).

L'huile volatile peut être présente dans la composition selon l'invention en une teneur allant de 0,1 % à 90 % en poids, par rapport au poids total de la composition, de préférence allant de 1 % à 70 % en poids, et préférentiellement allant de 5 % à 50 % en poids.

La composition peut comprendre, outre le polymère séquencé décrit précédemment selon l'invention, un polymère additionnel tel qu'un polymère filmogène. Selon la présente invention, on entend par "polymère filmogène", un polymère apte à former à lui seul ou en présence d'un agent auxiliaire de filmification, un film continu et adhérent sur un support, notamment sur les matières kératiniques.

Parmi les polymères filmogènes utilisables dans la composition de la présente invention, on peut citer les polymères synthétiques, de type radicalaire ou de type polycondensat, les polymères d'origine naturelle et leurs mélanges. Comme polymère filmogène, on peut citer en particulier les polymères acryliques, les polyuréthanes, les polyesters, les polyamides, les polyurées, les polymères cellulosiques comme la nitrocellulose.

La composition selon l'invention peut également comprendre au moins corps gras solides à température ambiante notamment choisi parmi les cires, les corps gras pâteux, les gommes et leurs mélanges. Ces corps gras peuvent être d'origine animale, végétale, minérale ou synthétique.

Par cire au sens de la présente invention, on entend un composé lipophile, solide à température ambiante (25 °C), à changement d'état solide/liquide réversible, ayant un point de fusion supérieur ou égal à 30 °C pouvant aller jusqu'à 120 °C.

En portant la cire à l'état liquide (fusion), il est possible de la rendre miscible aux huiles éventuellement présentes et de former un mélange homogène microscopiquement, mais en ramenant la température du mélange à la température ambiante, on obtient une recristallisation de la cire dans les huiles du mélange. Le point de fusion de la cire peut être mesuré à l'aide d'un calorimètre à balayage différentiel (D.S.C.), par exemple le calorimètre vendu sous la dénomination DSC 30 par la société METLER.

La cire peut également présenter une dureté allant de 0,05 MPa à 15 MPa, et de préférence allant de 6 MPa à 15 MPa . La dureté est déterminée par la mesure de la force en compression mesurée à 20 °C à l'aide du texturomètre vendu sous la dénomination TA-TX2i par la société RHEO, équipé d'un cylindre en inox d'un diamètre de 2 mm se déplaçant à la vitesse de mesure de 0,1 mm/s, et pénétrant dans la cire à une profondeur de pénétration de 0,3 mm.

Les cires peuvent être hydrocarbonées, fluorées et/ou siliconées et être d'origine végétale, minérale, animale et/ou synthétique. En particulier, les cires présentent une température de fusion supérieure à 25 °C et mieux supérieure à 45 °C.

Comme cire utilisable dans la composition de l'invention, on peut citer la cire d'abeilles, la cire de Carnauba ou de Candellila, la paraffine, les cires microcristallines, la cérésine ou l'ozokérite ; les cires synthétiques comme les cires de polyéthylène ou de Fischer Tropsch, les cires de silicones comme les alkyl ou alkoxy-diméticone ayant de 16 à 45 atomes de carbone.

Les gommes sont généralement des polydiméthylsiloxanes (PDMS) à haut poids moléculaire ou des gommes de cellulose ou des polysaccharides et les corps pâteux sont généralement des composés hydrocarbonés comme les lanolines et leurs dérivés ou encore des PDMS.

La nature et la quantité des corps solides sont fonction des propriétés mécaniques et des textures recherchées. A titre indicatif, la composition peut contenir de 0,1 à 50 % en poids de cires, par rapport au poids total de la composition et mieux de 1 à 30 % en poids.

La composition selon l'invention peut en outre comprendre une ou des matières colorantes choisies parmi les colorants hydrosolubles, et les matières colorantes pulvérulentes comme les pigments, les nacres, et les paillettes bien connues de l'homme du métier. Les matières colorantes peuvent être présentes, dans la composition, en une teneur allant de 0,01 % à 50 % en poids, par rapport au poids de la composition, de préférence de 0,01 % à 30 % en poids.

Par pigments, il faut comprendre des particules de toute forme, blanches ou colorées, minérales ou organiques, insolubles dans le milieu physiologique, destinées à colorer la composition.

Par nacres, il faut comprendre des particules de toute forme irisées, notamment produites par certains mollusques dans leur coquille ou bien synthétisées.

Les pigments peuvent être blancs ou colorés, minéraux et/ou organiques. On peut citer, parmi les pigments minéraux, le dioxyde de titane, éventuellement traité en surface, les oxydes de zirconium ou de cérium, ainsi que les oxydes de zinc, de fer (noir, jaune ou rouge) ou de chrome, le violet de manganèse, le bleu outremer, l'hydrate de chrome et le bleu ferrique, les poudres métalliques comme la poudre d'aluminium, la poudre de cuivre.

Parmi les pigments organiques, on peut citer le noir de carbone, les pigments de type D & C, et les laques à base de carmin de cochenille, de baryum, strontium, calcium, aluminium.

On peut également citer les pigments à effet tels les particules comportant un substrat organique ou minéral, naturel ou synthétique, par exemple le verre, les résines acrylique, le polyester, le polyuréthane, le polyéthylène téréphtalate, les céramiques ou les alumines, ledit substrat étant recouvert ou non de substances métalliques comme l'aluminium, l'or, l'argent, le platine, le cuivre, le bronze, ou d'oxydes métalliques comme le dioxyde de titane, l'oxyde de fer, l'oxyde de chrome et leurs mélanges.

Les pigments nacrés peuvent être choisis parmi les pigments nacrés blancs tels que le mica recouvert de titane, ou d'oxychlorure de bismuth, les pigments nacrés colorés tels que le mica titane recouvert avec des oxydes de fer, le mica titane recouvert avec notamment du bleu ferrique ou de l'oxyde de chrome, le mica titane recouvert avec un pigment organique du type précité ainsi que les pigments nacrés à base d'oxychlorure de bismuth.. On peut également utiliser les pigments interférentiels, notamment à cristaux liquides ou multicouches.

Les colorants hydrosolubles sont par exemple le jus de betterave, le bleu de méthylène.

La composition selon l'invention peut comprendre en outre une ou plusieurs charges, notamment en une teneur allant de 0,01 % à 50 % en poids, par rapport au poids total de la composition, de préférence allant de 0,01 % à 30 % en poids.

Par charges, il faut comprendre des particules de toute forme, incolores ou blanches, minérales ou de synthèse, insolubles dans le milieu de la composition quelle que soit la température à laquelle la composition est fabriquée. Ces charges servent notamment à modifier la rhéologie ou la texture de la composition.
Les charges peuvent être minérales ou organiques de toute forme, plaquettaires, sphériques ou oblongues, quelle que soit la forme cristallographique ( par exemple feuillet, cubique, hexagonale, orthorombique, etc). On peut citer le talc, le mica, la silice, le kaolin, les poudres de polyamide (Nylon®) (Orgasol® de chez Atochem), de poly-β-alanine et de polyéthylène, les poudres de polymères de tétrafluoroéthylène (Téflon®), la lauroyl-lysine, l'amidon, le nitrure de bore, les microsphères creuses polymériques telles que celles de chlorure de polyvinylidène/acrylonitrile comme l'Expancel® (Nobel Industrie), de copolymères d'acide acrylique (Polytrap® de la société Dow Corning) et les microbilles de résine de silicone (Tospearls® de Toshiba, par exemple), les particules de polyorganosiloxanes élastomères, le carbonate de calcium précipité, le carbonate et l'hydro-carbonate de magnésium, l'hydroxyapatite, les microsphères de silice creuses (Silica Beads® de Maprecos), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.

La composition peut comprendre, un milieu hydrophile comprenant de l'eau ou un mélange d'eau et de solvant (s) organique(s) hydrophile(s) comme les alcools et notamment les monoalcools inférieurs linéaires ou ramifiés ayant de 2 à 5 atomes de carbone comme l'éthanol, l'isopropanol ou le n-propanol, et les polyols comme la glycérine, la diglycérine, le propylène glycol, le sorbitol, le penthylène glycol, et les polyéthylène glycols, ou bien encore des éthers en $C_2$ et des aldéhydes en $C_2$-$C_4$ hydrophiles.
L'eau ou le mélange d'eau et de solvants organiques hydrophiles peut être présent dans la composition selon l'invention en une teneur allant de 0,1 % à 99 % en poids, par rapport au poids total de la composition, et de préférence de 10 % à 80 % en poids.

La composition selon l'invention peut également contenir des ingrédients couramment utilisés en cosmétique, tels que les vitamines, les épaississants, les oligo-éléments, les adoucissants, les séquestrants, les parfums, les agents alcalinisants ou acidifiants, les conservateurs, les filtres solaires, les tensioactifs, les anti-oxydants, les agents anti-chutes des cheveux, les agents anti-pelliculaires, les agents propulseurs, ou leurs mélanges.
Bien entendu, l'homme du métier veillera à choisir ce ou ces éventuels composés complémentaires, et/ou leur quantité, de manière telle que les propriétés avantageuses de la composition correspondante selon l'invention ne soient pas, ou substantiellement pas, altérées par l'adjonction envisagée.

La composition selon l'invention peut se présenter notamment sous forme de suspension, de dispersion, de solution, de gel, d'émulsion, notamment émulsion huile-dans-eau (H/E) ou eau-dans-huile (E/H), ou multiple (E/H/E ou polyol/H/E ou H/E/H), sous forme de crème, de mousse, de stick, de dispersion de vésicules notamment de lipides ioniques ou non, de lotion biphase ou multiphase, de spray, de poudre, de pâte, notamment de pâte souple (notamment de pâte ayant de viscosité dynamique à 25°C de l'ordre de 0,1 à 40 Pa.s sous une vitesse de cisaillement de 200 $s^{-1}$, après 10 minutes de mesure en géométrie cône/plan). La composition peut être anhydre, par exemple il peut s'agir d'un stick ou d'une pâte anhydre. La composition peut être une composition non rinçée.

L'homme du métier pourra choisir la forme galénique appropriée, ainsi que sa méthode de préparation, sur la base de ses connaissances générales, en tenant compte d'une part de la nature des constituants utilisés, notamment de leur solubilité dans le support, et d'autre part de l'application envisagée pour la composition.

Selon un autre aspect, l'invention concerne également un ensemble cosmétique comprenant :

i) un récipient délimitant au moins un compartiment, ledit récipient étant fermé par un élément de fermeture ; et
ii) une composition disposée à l'intérieur dudit compartiment, la composition étant conforme à l'une quelconque des revendications qui précèdent.

Le récipient peut être sous toute forme adéquate. Il peut être notamment sous forme d'un flacon, d'un tube,

d'un pot, d'un étui, d'une boite, d'un sachet ou d'un boîtier.

L'élément de fermeture peut être sous forme d'un bouchon amovible, d'un couvercle, d'un opercule, d'une bande déchirable, ou d'une capsule, notamment du type comportant un corps fixé au récipient et une casquette articulée sur le corps. Il peut être également sous forme d'un élément assurant la fermeture sélective du récipient, notamment une pompe, une valve, ou un clapet.

Le récipient peut être associé à un applicateur, notamment sous forme d'une brosse comportant un arrangement de poils maintenus par un fil torsadé. Une telle brosse torsadée est décrite notamment dans le brevet US 4 887 622. Il peut être également sous forme d'un peigne comportant une pluralité d'éléments d'application, obtenus notamment de moulage. De tels peignes sont décrits par exemple dans le brevet FR 2 796 529. L'applicateur peut être sous forme d'un pinceau, tel que décrit par exemple dans le brevet FR 2 722 380. L'applicateur peut être sous forme d'un bloc de mousse ou d'élastomère, d'un feutre, ou d'une spatule. L'applicateur peut être libre (houppette ou éponge) ou solidaire d'une tige portée par l'élément de fermeture, tel que décrit par exemple dans le brevet US 5 492 426. L'applicateur peut être solidaire du récipient, tel que décrit par exemple le brevet FR 2 761 959.

Le produit peut être contenu directement dans le récipient, ou indirectement. A titre d'exemple, le produit peut être disposé sur un support imprégné, notamment sous forme d'une lingette ou d'un tampon, et disposé (à l'unité ou plusieurs) dans une boîte ou dans un sachet. Un tel support incorporant le produit est décrit par exemple dans la demande WO 01/03538.

L'élément de fermeture peut être couplé au récipient par vissage. Alternativement, le couplage entre l'élément de fermeture et le récipient se fait autrement que par vissage, notamment via un mécanisme à baïonnette, par encliquetage, serrage, soudage, collage, ou par attraction magnétique. Par "encliquetage" on entend en particulier tout système impliquant le franchissement d'un bourrelet ou d'un cordon de matière par déformation élastique d'une portion, notamment de l'élément de fermeture, puis par retour en position non contrainte élastiquement de ladite portion après le franchissement du bourrelet ou du cordon.

Le récipient peut être au moins pour partie réalisé en matériau thermoplastique. A titre d'exemples de matériaux thermoplastiques, on peut citer le polypropylène ou le polyéthylène.
Alternativement, le récipient est réalisé en matériau non thermoplastique, notamment en verre ou en métal (ou alliage).

Le récipient peut être à parois rigides ou à parois déformables, notamment sous forme d'un tube ou d'un flacon tube.

Le récipient peut comprendre des moyens destinés à provoquer ou faciliter la distribution de la composition. A titre d'exemple, le récipient peut être à parois déformables de manière à provoquer la sortie de la composition en réponse à une surpression à l'intérieur du récipient, laquelle surpression est provoquée par écrasement élastique (ou non élastique) des parois du récipient. Alternativement, notamment lorsque le produit est sous forme d'un stick, ce dernier peut être entraîné par un mécanisme à piston. Toujours dans le cas d'un stick, notamment de produit de maquillage (rouge à lèvres, fond de teint, etc.), le récipient peut comporter un mécanisme, notamment à crémaillère, ou avec une tige filetée, ou avec une rampe hélicoïdale, et apte à déplacer un stick en direction de ladite ouverture. Un tel mécanisme est décrit par exemple dans le brevet FR 2 806 273 ou dans le brevet FR 2 775 566. Un tel mécanisme pour un produit liquide est décrit dans le brevet FR 2 727 609.

Le récipient peut être constitué d'un boîtier avec un fond délimitant au moins un logement contenant la composition, et un couvercle, notamment articulé sur le fond, et apte à recouvrir au moins en partie ledit fond. Un tel boîtier est décrit par exemple dans la demande WO 03/018423 ou dans le brevet FR 2 791 042.

Le récipient peut être équipé d'un essoreur disposé au voisinage de l'ouverture du récipient. Un tel essoreur permet d'essuyer l'applicateur et éventuellement, la tige dont il peut être solidaire. Un tel essoreur est décrit par exemple dans le brevet FR 2 792 618.

La composition peut être à la pression atmosphérique à l'intérieur du récipient (à température ambiante) ou pressurisée, notamment au moyen d'un gaz propulseur (aérosol). Dans ce dernier cas, le récipient est équipé d'une valve (du type de celles utilisées pour les aérosols).

Le contenu des brevets ou demandes de brevets cités précédemment sont incorporés par référence dans la présente demande.

L'invention est illustrée plus en détails par les exemples décrits ci-après.

**<u>Exemple 1</u> :**

**Préparation d'un polymère de poly(acrylate d'isobornyle/méthacrylate d'isobornyle /acrylate d'éthyl-2 hexyle)**

100 g d'isododécane sont introduits dans un réacteur de 1 litre, puis on augmente la température de façon à passer de la température ambiante (25°C) à 90°C en 1 heure.
On ajoute ensuite, à 90°C et en 1 heure, 105 g d'acrylate d'isobornyle, 105 g de méthacrylate d'isobornyl, 110 g d'isododécane et 1,8 g de 2.5- Bis(2-éthylhexanoylperoxy)-2.5-diméthylhexane (Trigonox® 141 d'Akzo Nobel).

Le mélange est maintenu 1 h 30 à 90°C.
On introduit ensuite au mélange précédent, toujours à 90°C et en 30 minutes, 90 g d'acrylate d'éthyle-2 hexyle, 90 g d'isododécane et 1,2 g de 2.5- Bis(2-éthylhexanoylperoxy)-2.5-diméthylhexane.
Le mélange est maintenu 3 heures à 90°C, puis l'ensemble est refroidi.
On obtient une solution à 50% de matière active en polymère dans l'isododécane.

On obtient un polymère comprenant une première séquence ou bloc poly(acrylate d'isobornyle/méthacrylate d'isobornyle) ayant une Tg de 110°C, une deuxième séquence polyacrylate d'éthyl-2 hexyle ayant une Tg de - 70°C et une séquence intermédiaire qui est un polymère statistique acrylate d'isobornyle/méthacrylate d'isobornyle/acrylate d'éthyl-2 hexyle.

Ce polymère présente une masse moyenne en poids de 103 900 et une masse moyenne en nombre de 21 300, soit un indice de polydispersité I de 4.89.

**Exemple 2:**

On a préparé un rouge à lèvres ayant la composition suivante :

| | |
|---|---|
| Polymère séquencé de l'exemple 1 à 50 % en poids dans l'isododécane | 65 g |
| Polyisobutylène hydrogéné (huile de Parleam) | 2,1 g |
| Octyldodécanol | 0,9 g |
| Huile de silicone phénylée (Dow Corning 556 C) | 27,8 g |
| Copolymère de polyvinyl pyrrolidone/Eicosène (Antaron V220 d'ISP) | 1,2 g |
| Pigments | 3 g |

On mélange l'octydodécanol, l'huile siliconée, l'huile de Parléam, l'acétate isobutyrate de saccharose, et le copolymère de polyvinyl pyrrolidone/Eicosène en chauffant à environ 60 °C. On réalise avec ce mélange un broyat pigmentaire des pigments en effectuant 3 passages du mélange à la broyeuse tri cylindres.

On mélange ensuite à température ambiante le broyat pigmentaire, l'isododécane, le polymère séquencé, puis on introduit au final la silice. La formule est ensuite introduite dans une bouillote étanche.

On a ensuite évalué les propriétés de non transfert du film de maquillage obtenu avec ce rouge à lèvres, en utilisant le protocole suivant :

On préchauffe un support (rectangle de 40 mm X 70 mm et d'épaisseur 3 mm) de mousse de polyéthylène adhésif sur une des faces ayant une densité de 33 kg/m3 (vendue sous la dénomination RE40X70EP3 de la société JOINT TECHNIQUE LYONNAIS IND) sur une plaque chauffante maintenue à la température de 40 °C pour que la surface du support soit maintenue à une température de 33 °C ± 1 °C.

Tout en laissant le support sur la plaque chauffante, on applique la composition sur toute la surface non adhésive du support en l'étalant à l'aide d'un pinceau pour obtenir un dépôt de la composition d'environ 15 µm puis on laisse sécher pendant 30 minutes.

Après séchage, le support est collé par sa face adhésive sur une enclume d'un diamètre de 20 mm et munie d'un pas de vis. L'ensemble support/dépôt est ensuite découpé à l'aide d'un emporte- pièce d'un diamètre de 18 mm. L'enclume est ensuite vissée sur une presse (STATIF MANUEL IMADA SV-2 de la société SOMECO) équipée d'un dynanomètre (IMADA DPS-20 de la société SOMECO).

Un papier blanc pour photocopieuse de 80g/m2 est placé sur le socle de la presse puis on presse l'ensemble support/dépôt sur le papier à une pression de 2,5 kg pendant 30 secondes. Après retrait de l'ensemble support/dépôt, une partie du dépôt a transféré sur le papier. On mesure alors la couleur du dépôt transféré sur le papier à l'aide d'un colorimètre MINOLTA CR300 , la couleur étant caractérisée par les paramètres colorimétriques L*, a*, b* . On détermine les paramètres colorimétriques $L^*_0$, $a^*_0$, $b^*_0$ de la couleur du papier nu utilisé.

On détermine alors la différence de couleur ΔE1 entre la couleur du dépôt transféré par rapport à la couleur du papier nu par la relation suivante.

$$\Delta E1 = \sqrt{(L^*-L_o^*)^2 + (a^* - a_o^*)^2 + (b^* - b_o^*)^2}$$

Par ailleurs, on prépare une référence de transfert total en appliquant la composition directement sur un papier identique à celui utilisé précédemment, à la température ambiante (25 °C), en étalant la composition à l'aide d'un pinceau et pour obtenir un dépôt de la composition d'environ 15 µm puis on laisse sécher pendant 30 minutes à la température ambiante (25 °C). Après séchage, on mesure directement les paramètres colorimétriques L*', a*', b*' de la couleur du dépôt mis sur le papier, correspondant à la couleur de référence de transfert total. On détermine les paramètres colorimétriques $L^{*'}_0$, $a^{*'}_0$, $b^{*'}_0$ de la couleur du papier nu utilisé.

On détermine alors la différence de couleur ΔE2 entre la couleur de référence de transfert total par rapport à la couleur du papier nu par la relation suivante.

$$\Delta E2 = \sqrt{(L^{*'}-L_o^{*'})^2 + (a^{*'} - a_o^{*'})^2 + (b^{*'} - b_o^{*'})^2}$$

Le transfert de la composition, exprimé en pourcentage, est égal au rapport :

$$100 \times \Delta E1 / \Delta E2$$

La mesure est effectuée sur 4 supports à la suite et la valeur de transfert correspond à la moyenne des 4 mesures obtenues avec les 4 supports.

Le rouge à lèvres de l'exemple 2 forme un film ayant un transfert de 18 % ± 2 %.

**Exemple 3 :**

On a préparé un rouge à lèvres, ne faisant pas partie de l'invention, ayant la composition suivante :

| | |
|---|---|
| Polymère séquencé de l'exemple 1 à 50 % en poids dans l'isododécane | 65 g |
| Polyisobutylène hydrogéné (huile de Parleam) | 27,8 g |
| Octyldodécanol | 0,9 g |
| Huile de silicone phénylée (Dow Corning 556 C) | 2,1 g |
| Copolymère de polyvinyl pyrrolidone/Eicosène (Antaron V220 d'ISP) | 1,2 g |
| Pigments | 3 g |

Ce rouge à lèvres, par rapport au rouge à lèvres de l'exemple 2, contient un taux d'huile de silicone non volatile bien plus faible (2,1 % au lieu de 27,8 %) et un taux d'huile de Parléam plus élevé (27,8 % au lieu de 2,1 %).

Le film obtenu avec ce rouge à lèvres a un non transfert, mesure selon le m^me protocole décrit dans l'exemple 2, de 54 % ± 1 %. Ce rouge à lèvres a donc des propriétés de non transfert moins bonne que celles du rouge à lèvres de l'exemple 2 selon l'invention.

## Revendications

1. Composition cosmétique comprenant un polymère séquencé et un milieu liquide organique cosmétiquement acceptable contenant une phase grasse liquide non volatile, ladite phase grasse liquide non volatile comprenant au moins 30 % en poids, par rapport au poids total de la phase grasse liquide non volatile, d'huile siliconée non volatile, ladite composition étant **caractérisée par le fait que** le polymère séquencé est un polymère éthylénique linéaire filmogène, comprenant au moins une première séquence ayant une température de transition vitreuse (Tg) supérieure ou égale à 40°C issue en totalité ou en partie de un ou plusieurs monomères dont l'homopolymère a une température de transition vitreuse supérieure ou égale à 40°C et au moins une deuxième séquence ayant une température de transition vitreuse inférieure ou égale à 20°C issue en totalité ou en partie de un ou plusieurs

monomères dont l'homopolymère a une température de transition vitreuse inférieure ou égale à 20°C, les monomères dont l'homopolymère a une température de transition vitreuse supérieure ou égale à 40°C étant choisis parmi les monomères suivants :

- les méthacrylates d'isobornyle
- les acrylates d'isobornyle,
- et leurs mélanges,

et les monomères dont l'homopolymère a une température de transition vitreuse inférieure ou égale à 20°C étant choisis parmi les acrylates de formule $CH_2 = CHCOOR_3$, $R_3$ représentant un groupe alkyle non substitué en $C_1$ à $C_{12}$, linéaire ou ramifié, à l'exception du groupe tertiobutyle, dans lequel se trouve(nt) éventuellement intercalé(s) un ou plusieurs hétéroatomes choisis parmi O, N, S.

2. Composition selon la revendication 1 **caractérisée en ce que** le polymère séquencé est exempt de styrène.

3. Composition selon la revendication 1 ou 2 **caractérisée en ce que** le polymère séquencé est non élastomère.

4. Composition selon la revendication précédente **caractérisée en ce que** les première et seconde séquences sont incompatibles l'une avec l'autre.

5. Composition selon l'une quelconque des revendications qui précèdent **caractérisée en ce que** le polymère séquencé a un indice de polydispersité I supérieur à 2.

6. Composition selon la revendication précédente, **caractérisée par le fait que** la proportion de la première séquence va de 20 à 90% en poids du polymère, mieux de 30 à 80% et encore mieux de 50 à 70%.

7. Composition selon la revendication précédentes, **caractérisée par le fait que** la proportion de la deuxième séquence ayant une Tg inférieure ou égale à 20°C va de 5 à 75% en poids du polymère, mieux de 15 à 50% et encore mieux de 25 à 45%.

8. Composition selon l'une quelconque des revendications précédentes, **caractérisée par le fait que** la séquence ayant une Tg supérieure ou égale à 40°C est issue en totalité ou en partie de un ou plusieurs monomères dont l'homopolymère a une température de transition vitreuse allant de 40 à 150 °C, de préférence supérieure ou égale à 50°C, notamment allant de 50°C à 120°C, et préférentiellement supérieure ou égale à 60°C, notamment allant de 60°C à 120°C.

9. Composition selon la revendication précédente, **caractérisée par le fait que** la séquence ayant une Tg supérieure ou égale à 40 °C est un copolymère issu de monomères dont l'homopolymère a une température de transition vitreuse supérieure ou égale à 40°C.

10. Composition selon l'une quelconque des revendications précédentes, **caractérisée par le fait que** la séquence ayant une Tg supérieure ou égale à 40°C est un homopolymère.

11. Composition selon l'une quelconque des revendications précédentes, **caractérisée par le fait que** la séquence ayant une Tg inférieure ou égale à 20°C est issue en totalité ou en partie de un ou plusieurs monomères dont l'homopolymère a une température de transition vitreuse allant de -100 à 20°C, de préférence inférieure ou égale à 15°C, notamment allant de -80°C à 15°C et préférentiellement inférieure ou égale à 10°C, notamment allant de -50°C à 0°C.

12. Composition selon la revendication précédente, **caractérisée par le fait que** les monomères dont l'homopolymère a une température de transition vitreuse inférieure ou égale à 20°C sont choisis parmi les acrylates d'alkyle dont la chaîne alkyle comprend de 1 à 10 atomes de carbone, à l'exception du groupe tertiobutyle

13. Composition selon l'une quelconque des revendications précédentes, **caractérisée par le fait que** la séquence ayant une température de transition vitreuse inférieure ou égale à 20°C est un homopolymère.

14. Composition selon l'une quelconque des revendications précédentes, **caractérisée par le fait que** la première séquence et/ou la deuxième séquence comprend au moins un monomère additionnel.

**15.** Composition selon la revendication précédente, **caractérisée par le fait que** le monomère additionnel est choisi parmi les monomères hydrophiles, les monomères à insaturation éthylénique comprenant un ou plusieurs atomes de silicium et leurs mélanges.

**16.** Composition selon la revendication 14 ou 115, **caractérisée par le fait que** le monomère additionnel est choisi parmi :

- les monomères à insaturation(s) éthylénique(s) comprenant au moins une fonction acide carboxylique ou sulfonique
- les méthacrylates de formule $CH_2 = C(CH_3)\text{-}COOR_6$
dans laquelle $R_6$ représente un groupe alkyle linéaire ou ramifié, contenant de 1 à 4 atomes de carbone, ledit groupe alkyle étant substitué par un ou plusieurs substituants choisis parmi le groupe hydroxyle et les atomes d'halogènes,
- les méthacrylates de formule $CH_2 = C(CH_3)\text{-}COOR_9$,
$R_9$ représentant un groupe alkyle en $C_6$ à $C_{12}$ linéaire ou ramifié, dans lequel se trouve(nt) éventuellement intercalé(s) un ou plusieurs hétéroatomes choisis parmi 0, N et S, ledit groupe alkyle étant substitué par un ou plusieurs substituants choisis parmi le groupe hydroxyle et les atomes d'halogènes ;
- les acrylates de formule $CH_2 = CHCOOR_{10}$,
$R_{10}$ représentant un groupe alkyle en $C_1$ à $C_{12}$ linéaire ou ramifié substitué par un ou plusieurs substituants choisis parmi le groupe hydroxyle et les atomes d'halogène, ou $R_{10}$ représente un alkyle($C_1$-$C_{12}$)-O-POE (polyoxyéthylène) avec répétition du motif oxyéthylène de 5 à 30 fois, ou $R_{10}$ représente un groupement polyoxyéthylèné comprenant de 5 à 30 motifs d'oxyde d'éthylène
- les monomères à insaturation(s) éthylénique(s) comprenant au moins une fonction amine tertiaire,
et leurs mélanges.

**17.** Composition selon l'une quelconque des revendications 14 à 16, **caractérisée par le fait que** le ou les monomères additionnels sont choisis parmi l'acide acrylique, l'acide méthacrylique, le méthacrylate de trifluoroéthyle et leurs mélanges.

**18.** Composition selon l'une des revendications 14 à 17, **caractérisée par le fait que** le ou les monomères additionnel (s) représente(nt) de 1 à 30% en poids du poids total des première et/ou deuxième séquences.

**19.** Composition selon l'une des revendications précédentes, **caractérisée par le fait que** chacune des première et deuxième séquence comprend au moins un monomère choisi parmi les esters d'acide (méth)acrylique, et éventuellement au moins un monomère choisi parmi l'acide (méth)acrylique, et leurs mélanges.

**20.** Composition selon l'une des revendications précédentes, **caractérisée par le fait que** chacune des première et deuxième séquence est issue en totalité d'au moins un monomère choisi parmi les esters d'acide (méth)acrylique, et éventuellement d'au moins un monomère choisi parmi l'acide (méth)acrylique, et leurs mélanges

**21.** Composition selon l'une quelconque des revendications précédentes, **caractérisée par le fait que** le polymère séquencé est exempt de monomère styrénique.

**22.** Composition selon l'une des revendications précédentes, **caractérisée par le fait que** les première et deuxième séquences sont telles que l'écart entre les températures de transition vitreuse (Tg) des première et deuxième séquences est supérieur à 10°C, de préférence supérieur à 20°C, préférentiellement supérieur à 30°C, et plus préférentiellement supérieur à 40°C.

**23.** Composition selon l'une des revendications précédentes, **caractérisée par le fait que** la séquence intermédiaire a une température de transition vitreuse comprise entre les températures de transition vitreuse des première et deuxième séquences.

**24.** Composition selon l'une des revendications précédentes, **caractérisée par le fait que** le polymère séquencé a un indice de polydispersité supérieur ou égal à 2,5, de préférence supérieure ou égal à 2,8, de préférence compris entre 2,8 et 6.

**25.** Composition selon l'une des revendications précédentes, **caractérisée par le fait que** le polymère séquencé a une masse moyenne en poids (Mw) inférieure ou égale à 300 000, de préférence allant de 35 000 à 200 000, et

mieux allant de 45 000 à 150 000.

**26.** Composition selon l'une des revendications précédentes, **caractérisée par le fait que** le polymère séquencé a masse moyenne en nombre (Mn) est inférieure ou égale à 70 000, de préférence allant de 10 000 à 60 000, et mieux allant de 12 000 à 50 000.

**27.** Composition selon l'une des revendications précédentes, **caractérisée par le fait que** le polymère séquencé n'est pas soluble à une teneur en matière active d'au moins 1 % en poids dans l'eau ou dans un mélange d'eau et de monoalcools inférieurs linéaires ou ramifiés ayant de 2 à 5 atomes de carbone, sans modification de pH, à température ambiante (25°C).

**28.** Composition selon l'une quelconque des revendications précédentes, **caractérisée par le fait que** le polymère séquence est présent en une teneur allant de 0,1 % à 90 % en poids, par rapport au poids total de la composition, de préférence allant de 0,5 % à 50 % en poids, et préférentiellement allant de 0,5 % à 30 % en poids.

**29.** Composition selon l'une quelconque des revendications précédentes, **caractérisée par le fait que** l'huile non volatile siliconée est choisie parmi les polydiméthylsiloxanes (PDMS) non volatils ; les polydiméthylsiloxanes comportant des groupements alkyle, alcoxy ou phényle, pendant ou en bout de chaîne siliconée, groupements ayant de 2 à 24 atomes de carbone ; les silicones phénylées; les polysiloxanes modifiés par des acides gras (notamment en $C_8$-$C_{20}$), des alcools gras (notamment en $C_8$-$C_{20}$) ou des polyoxyalkylènes (notamment polyoxyéthyléne et/ou polyoxypropylène) ; les silicones aminées ; les silicones à groupement hydroxyles ; les silicones fluorés comportant un groupement fluoré pendant ou en bout de chaîne siliconée ayant de 1 à 12 atomes de carbone dont tout ou partie des hydrogène sont substitués par des atomes de fluor ; et leurs mélanges.

**30.** Composition selon l'une quelconque des revendications précédentes, **caractérisée par le fait que** l'huile siliconée non volatile est choisie parmi les huiles siliconées phénylées non volatiles.

**31.** Composition selon la revendication précédente, **caractérisée par le fait que** l'huile siliconée phénylée non volatile est choisie parmi les phényl trimethicones, les phényl dimethicones, les phényl triméthylsiloxy diphénylsiloxanes, les diphényl diméthicones, les diphényl méthyldiphényl trisiloxanes, et leurs mélanges.

**32.** Composition selon l'une des revendications 30 ou 31, **caractérisée par le fait que** l'huile siliconée phénylée non volatile peut être choisie parmi les silicones phénylées de formule (VI) suivante :

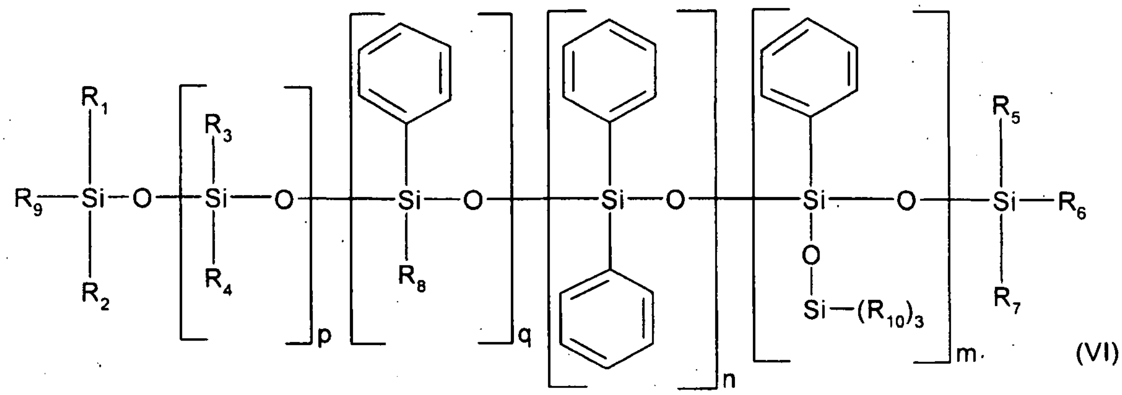

(VI)

dans laquelle

- R1 à R10, indépendamment les uns des autres, sont des radicaux hydrocarbonés, saturés ou insaturés, linéaires, cycliques ou ramifiés, en C1-C30,
- m, n, p et q sont, indépendamment les uns des autres, des nombres entiers compris entre 0 et 900, sous réserve que la somme 'm+n+q' est différente de 0.

**33.** Composition selon la revendication précédente, **caractérisée par le fait que** :

- la somme 'm+n+q' est comprise entre 1 et 100.
- la somme 'm+n+p+q' est comprise entre 1 et 900, encore mieux entre 1 et 800.
- q est égal à 0.

**34.** Composition selon la revendication 32 ou 33, **caractérisée par le fait que** l'huile siliconée phénylée de formule (VI) a une viscosité à 25°C comprise entre 5 et 1500 $mm^2/s$ (soit 5 à 1500 cSt), de préférence comprise entre 5 et 1000 $mm^2/s$ (soit 5 à 1000 cSt).

**35.** Composition selon la revendication 30 ou 31, **caractérisée par le fait que** l'huile siliconée phénylée non volatile est choisie parmi les silicones phénylées de formule (VII) suivante :

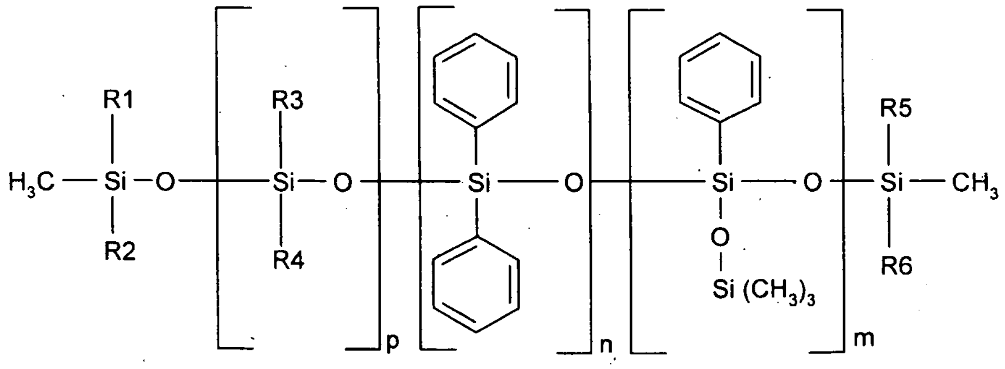
(VII)

dans laquelle :

- R1 à R6, indépendamment les uns des autres, sont des radicaux hydrocarbonés, saturés ou insaturés, linéaires cycliques ou ramifiés, en C1-C30,
- m, n et p sont, indépendamment les uns des autres, des nombres entiers compris entre 0 et 100, sous réserve que la somme 'n + m' est comprise entre 1 et 100.

**36.** Composition selon la revendication précédente, **caractérisée par le fait que** R1 à R6, indépendamment les uns des autres, représentent un radical hydrocarboné saturé, linéaire ou ramifié, en C1-C30, notamment en C1-C12, et en particulier un radical méthyle, éthyle, propyle ou butyle.

**37.** Composition selon la revendication 35 ou 36, **caractérisée par le fait que** R1 à R6 sont identiques, et sont un radical méthyle.

**38.** Composition selon l'une quelconque des revendications 35 à 37, **caractérisée par le fait que** m=1 ou 2 ou 3, et/ou n=0 et/ou p=0 ou 1.

**39.** Composition selon la revendication précédente, **caractérisée par le fait que** l'huile siliconée phénylée a un poids moléculaire en poids compris entre 500 et 10 000.

**40.** Composition selon l'une quelconque des revendications précédentes, **caractérisée par le fait que** l'huile siliconée non volatile est présente en une teneur allant de 30 % à 95 % en poids, par rapport au poids total de la phase grasse liquide non volatile, de préférence allant de 40 % à 85 % en poids, et préférentiellement allant de 50 % à 80 % en poids.

**41.** Composition selon l'une quelconque des revendications précédentes, **caractérisée par le fait que** l'huile siliconée non volatile est présente en une teneur allant de 0,1 % à 70 % en poids, par rapport au poids total de la composition, de préférence allant de 1 % à 50 % en poids, et préférentiellement allant de 1 % à 30 % en poids.

**42.** Composition selon l'une quelconque des revendications précédentes, **caractérisée par le fait qu'**elle comprend une huile non volatile non siliconée.

**43.** Composition selon la revendication précédente, **caractérisée par le fait que** l'huile non volatile non siliconée est choisie parmi l'huile de paraffine, le squalane, le polyisobutylène hydrogéné, le perhydrosqualène, l'huile de vison,

de tortue, de soja, l'huile d'amande douce, de calophyllum, de palme, de pépins de raisin, de sésame, de maïs, d'arara, de colza, de tournesol, de coton, d'abricot, de ricin, d'avocat, de jojoba, d'olive ou de germes de céréales, le myristate d'isopropyle, le palmitate d'isopropyle, le stéarate de butyle, le laurate d'hexyle, l'adipate de diisopropyle, l'isononanoate d'isononyle, le palmitate de 2-éthyl-hexyle, le laurate de 2-hexyl-décyle, le palmitate de 2-octyl-décyle, le myristate ou le lactate de 2-octyl-dodécyle, le succinate de di(2-éthyl hexyle), le malate de diisostéaryle, le triisostéarate de glycérine ou de diglycérine, l'acide myristique, l'acide palmitique, l'acide stéarique, l'acide béhénique, l'acide oléique, l'acide linoléique, l'acide linolénique ou l'acide isostéarique ; les alcools gras en $C_{16}$- $C_{22}$ ; et leurs mélanges.

**44.** Composition selon la revendication 42 ou 43, **caractérisée par le fait que** l'huile non volatile non siliconée est présente en une teneur allant de 0,1 % à 70 % en poids, par rapport au poids total de la phase grasse liquide non volatile, de préférence allant de 0,5 % à 60 % en poids, et préférentiellement allant de 1 % à 50 % en poids.

**45.** Composition selon l'une quelconque des revendications 42 à 44, **caractérisée par le fait que** l'huile non volatile non siliconée est présente en une teneur allant de 0,1 % à 60 % en poids, par rapport au poids total de la composition, de préférence allant de 0,5 % à 30 % en poids, et préférentiellement allant de 1 % à 20 % en poids.

**46.** Composition selon l'une quelconque des revendications précédentes, **caractérisée par le fait que** qu'elle comprend un huile volatile.

**47.** Composition selon l'une quelconque des revendications précédentes, **caractérisée par le fait qu'**elle comprend un huile volatile choisie parmi l'octaméthylcyclotétrasiloxane, le décaméthylcyclopentasiloxane, le dodécaméthylcyclohexasiloxane, l'heptaméthyl-hexyltrisiloxane, l'heptaméthyloctyltrisiloxane, l'octaméthyltrisiloxane, le décaméthyltétrasiloxane, l'isododécane, l'isodécane, l'isohexadécane.

**48.** Composition selon la revendication 46 ou 47 **caractérisée par le fait que** l'huile volatile est présente en une teneur allant de 0,1 % à 90 % en poids, par rapport au poids total de la composition, de préférence allant de 1 % à 70 % en poids, et préférentiellement allant de 5 % à 50 % en poids.

**49.** Composition selon l'une quelconque des revendications précédentes, **caractérisée par le fait qu'**elle comprend au moins un corps gras solides à température ambiante choisi parmi les cires, les corps gras pâteux, les gommes et leurs mélanges.

**50.** Composition selon l'une quelconque des revendications précédentes, **caractérisée par le fait qu'**elle contient de 0,1 à 50 % en poids de cires, par rapport au poids total de la composition, et de préférence de 1 à 30 % en poids.

**51.** Composition selon l'une quelconque des revendications précédentes, **caractérisée par le fait qu'**elle comprend une matière colorante.

**52.** Composition selon l'une quelconque des revendications précédentes, **caractérisée par le fait qu'**elle comprend un ingrédient cosmétique choisi parmi les polymères filmogènes additionnels, les vitamines, les épaississants, les oligo-éléments, les adoucissants, les séquestrants, les parfums, les agents alcalinisants ou acidifiants, les conservateurs, les filtres solaires, les tensioactifs, les antioxydants, les agents anti-chutes des cheveux, les agents antipelliculaires, les agents propulseurs, ou leurs mélanges.

**53.** Composition cosmétique selon l'une quelconque des revendications précédentes, **caractérisée par le fait qu'**elle se présente sous forme de suspension, de dispersion, de solution, de gel, d'émulsion, notamment émulsion huile-dans-eau (H/E) ou eau-dans-huile (E/H), ou multiple (E/H/E ou polyol/H/E ou H/E/H), sous forme de crème, de pâte, de mousse, de dispersion de vésicules notamment de lipides ioniques ou non, de lotion biphase ou multiphase, de spray, de stick.

**54.** Composition cosmétique selon l'une quelconque des revendications précédentes, **caractérisée par le fait qu'**elle se présente sous forme anhydre.

**55.** Composition cosmétique selon l'une quelconque des revendications précédentes, **caractérisée par le fait qu'**il s'agit d'une composition de maquillage ou de soin des matières kératiniques.

**56.** Composition selon l'une quelconque des revendications précédentes, **caractérisée par le fait que** la composition

est un produit de maquillage des lèvres.

**57.** Ensemble cosmétique comprenant :

a) un récipient délimitant au moins un compartiment, ledit récipient étant fermé par un élément de fermeture; et
b) une composition disposée à l'intérieur dudit compartiment, la composition étant conforme à l'une quelconque des revendications qui précèdent.

**58.** Ensemble cosmétique selon la revendication 57, **caractérisé par le fait que** le récipient est formé, au moins pour partie, en au moins un matériau thermoplastique.

**59.** Ensemble cosmétique selon la revendication 57, **caractérisé par le fait que** le récipient est formé, au moins pour partie, en au moins un matériau non thermoplastique, notamment en verre ou en métal.

**60.** Ensemble selon l'une quelconque des revendications 57 à 59, **caractérisé par le fait que**, en position fermée du récipient, l'élément de fermeture est vissé sur le récipient.

**61.** Ensemble selon l'une quelconque des revendications 57 à 59, **caractérisé par le fait que**, en position fermée du récipient, l'élément de fermeture est couplé au récipient autrement que par vissage, notamment par encliquetage, collage, ou soudage.

**62.** Ensemble selon l'une quelconque des revendications 57 à 61, **caractérisé par le fait que** la composition est sensiblement à la pression atmosphérique à l'intérieur du compartiment.

**63.** Ensemble selon l'une quelconque des revendications 57 à 61, **caractérisé par le fait que** la composition est pressurisée à l'intérieur du récipient.

**64.** Procédé cosmétique de maquillage ou de soin des matières kératiniques comprenant l'application sur les matières kératiniques d'une composition cosmétique de la composition cosmétique selon l'une des revendications 1 à 63.

**65.** Utilisation d'une composition selon l'une des revendications 1 à 63 pour obtenir un dépôt sur les matières kératiniques, en particulier sur la peau ou les lèvres, ayant des propriétés de sans transfert et confortable au cours du temps.

## Claims

**1.** Cosmetic composition comprising a block polymer and a cosmetically acceptable organic liquid medium containing a non-volatile liquid fatty phase, the said non-volatile liquid fatty phase comprising at least 30% by weight, relative to the total weight of the non-volatile liquid fatty phase, of non-volatile silicone oil, the said composition being **characterized in that** the block polymer is a film-forming linear ethylenic polymer, comprising at least one first block with a glass transition temperature (Tg) of greater than or equal to 40°C, totally or partially derived from one or more monomers whose homopolymer has a glass transition temperature of greater than or equal to 40°C, and at least one second block with a glass transition temperature of less than or equal to 20°C, totally or partially derived from one or more monomers whose homopolymer has a glass, transition temperature of less than or equal to 20°C,
the monomers whose homopolymer has a glass transition temperature of greater than or equal to 40°C being chosen from the following monomers:

- isobornyl methacrylates,
- isobornyl acrylates,
- and mixtures thereof,

and the monomers whose homopolymer has a glass transition temperature of less than or equal to 20°C being chosen from the acrylates of formula $CH_2$=$CHCOOR_3$,
$R_3$ representing a linear or branched, unsubstituted $C_1$ to $C_{12}$ alkyl group, with the exception of the ter-butyl group, in which is (are) optionally intercalated one or more heteroatoms chosen from O, N and S.

**2.** Composition according to Claim 1, **characterized in that** the block polymer is free of styrene.

**3.** Composition according to Claim 1 or 2, **characterized in that** the block polymer is non-elastomeric.

4. Composition according to the preceding claim, **characterized in that** the first and second blocks are mutually incompatible.

5. Composition according to any one of the preceding claims, **characterized in that** the block polymer has a polydispersity index I of greater than 2.

6. Composition according to the preceding claim, **characterized in that** the proportion of the first block ranges from 20% to 90%, better still from 30% to 80% and even better still from 50% to 70% by weight of the polymer.

7. Composition according to the preceding claim, **characterized in that** the proportion of the second block with a Tg of less than or equal to 20°C ranges from 5% to 75%, better still from 15% to 50% and even better still from 25% to 45% by weight of the polymer.

8. Composition according to any one of the preceding claims, **characterized in that** the block with a Tg of greater than or equal to 40°C is totally or partially derived from one or more monomers whose homopolymer has a glass transition temperature ranging from 40 to 150°C, preferably greater than or equal to 50°C, especially ranging from 50°C to 120°C and preferably greater than or equal to 60°C, especially ranging from 60°C to 120°C.

9. Composition according to the preceding claim, **characterized in that** the block with a Tg of greater than or equal to 40°C is a copolymer derived from monomers whose homopolymer has a glass transition temperature of greater than or equal to 40°C.

10. Composition according to any one of the preceding claims, **characterized in that** the block with a Tg of greater than or equal to 40°C is a homopolymer.

11. Composition according to any one of the preceding claims, **characterized in that** the block with a Tg of less than or equal to 20°C is totally or partially derived from one or more monomers whose homopolymer has a glass transition temperature ranging from -100 to 20°C, preferably less than or equal to 15°C, especially ranging from -80°C to 15°C and preferentially less than or equal to 10°C, especially ranging from -50°C to 0°C.

12. Composition according to the preceding claim, **characterized in that** the monomers whose homopolymer has a glass transition temperature of less than or equal to 20°C are chosen from alkyl acrylates whose alkyl chain contains from 1 to 10 carbon atoms, with the exception of the tert-butyl group.

13. Composition according to any one of the preceding claims, **characterized in that** the block with a glass transition temperature of less than or equal to 20°C is a homopolymer.

14. Composition according to any one of the preceding claims, **characterized in that** the first block and/or the second block comprise(s) at least one additional monomer.

15. Composition according to the preceding claim, **characterized in that** the additional monomer is chosen from hydrophilic monomers, monomers containing ethylenic unsaturation comprising one or more silicon atoms, and mixtures thereof.

16. Composition according to Claim 14 or 15, **characterized in that** the additional monomer is chosen from:

- ethylenically unsaturated monomers comprising at least one carboxylic or sulfonic acid function,
- methacrylates of formula $CH_2 = C(CH_3)\text{-}COOR_6$

in which $R_6$ represents a linear or branched alkyl group containing from 1 to 4 carbon atoms, the said alkyl group being substituted with one or more substituents chosen from hydroxyl groups and halogen atoms,

- methacrylates of formula $CH_2 = C(CH_3)\text{-}COOR_9$,
$R_9$ representing a linear or branched $C_6$ to $C_{12}$ alkyl group in which one or more heteroatoms chosen from O, N and S is (are) optionally intercalated, the said alkyl group being substituted with one or more substituents chosen from hydroxyl groups and halogen atoms;
- acrylates of formula $CH_2 = CHCOOR_{10}$,
$R_{10}$ representing a linear or branched $C_1$ to $C_{12}$ alkyl group substituted with one or more substituents chosen

from hydroxyl groups and halogen atoms, or $R_{10}$ represents a $C_1$ to $C_{12}$ alkyl-O-POE (polyoxyethylene) with repetition of the oxyethylene unit 5 to 30 times, or $R_{10}$ represents a polyoxyethylenated group comprising from 5 to 30 ethylene oxide units;
- ethylenically unsaturated monomers comprising at least one tertiary amine function,
and mixtures thereof.

**17.** Composition according to any one of Claims 14 to 16, **characterized in that** the additional monomer(s) is (are) chosen from acrylic acid, methacrylic acid and trifluoroethyl methacrylate, and mixtures thereof.

**18.** Composition according to one of Claims 14 to 17, **characterized in that** the additional monomer(s) represent(s) from 1% to 30% by weight relative to the total weight of the first and/or second blocks.

**19.** Composition according to one of the preceding claims, **characterized in that** each of the first and second blocks comprises at least one monomer chosen from (meth)acrylic acid esters, and optionally at least one monomer chosen from (meth)acrylic acid, and mixtures thereof.

**20.** Composition according to one of the preceding claims, **characterized in that** each of the first and second blocks is totally derived from at least one monomer chosen from (meth)acrylic acid esters, and optionally from at least one monomer chosen from (meth)acrylic acid, and mixtures thereof.

**21.** Composition according to any one of the preceding claims, **characterized in that** the block polymer is free of styrene monomer.

**22.** Composition according to one of the preceding claims, **characterized in that** the first and second blocks are such that the difference between the glass transition temperatures (Tg) of the first and second blocks is greater than 10°C, preferably greater than 20°C, preferentially greater than 30°C and more preferentially greater than 40°C.

**23.** Composition according to one of the preceding claims, **characterized in that** the intermediate block has a glass transition temperature that is between the glass transition temperatures of the first and second blocks.

**24.** Composition according to one of the preceding claims, **characterized in that** the block polymer has a polydispersity index of greater than or equal to 2.5, preferably greater than or equal to 2.8 and preferably between 2.8 and 6.

**25.** Composition according to one of the preceding claims, **characterized in that** the block polymer has a weight-average mass (Mw) of less than or equal to 300 000, preferably ranging from 35 000 to 200 000 and better still ranging from 45 000 to 150 000.

**26.** Composition according to one of the preceding claims, **characterized in that** the block polymer has a number-average mass (Mn) of less than or equal to 70 000, preferably ranging from 10 000 to 60 000 and better still ranging from 12 000 to 50 000.

**27.** Composition according to one of the preceding claims, **characterized in that** the block polymer is not soluble at an active material content of at least 1% by weight in water or in a mixture of water and of linear or branched lower monoalcohols containing from 2 to 5 carbon atoms, without pH modification, at room temperature (25°C).

**28.** Composition according to any one of the preceding claims, **characterized in that** the block polymer is present in a content ranging from 0.1% to 90% by weight, preferably ranging from 0.5% to 50% by weight and preferentially ranging from 0.5% to 30% by weight, relative to the total weight of the composition.

**29.** Composition according to any one of the preceding claims, **characterized in that** the non-volatile silicone oil is chosen from non-volatile polydimethylsiloxanes (PDMSs); polydimethylsiloxanes comprising alkyl, alkoxy or phenyl groups, pendent or at the end of a silicone chain, these groups containing from 2 to 24 carbon atoms; phenyl silicones; polysiloxanes modified with fatty acids (especially of $C_8$-$C_{20}$) fatty alcohols (especially of $C_8$-$C_{20}$) or poly-oxyalkylenes (especially polyoxyethylene and/or polyoxypropylene); amino silicones; silicones containing hydroxyl groups; fluoro silicones comprising a fluoro group that is pendent or at the end of a silicone chain, containing from 1 to 12 carbon atoms, some or all of the hydrogens of which are replaced with fluorine atoms; and mixtures thereof.

**30.** Composition according to any one of the preceding claims, **characterized in that** the non-volatile silicone oil is

chosen from non-volatile phenyl silicone oils.

**31.** Composition according to the preceding claim, **characterized in that** the non-volatile phenyl silicone oil is chosen from phenyl trimethicones, phenyl dimethicones, phenyltrimethylsiloxydiphenylsiloxanes, diphenyl dimethicones and diphenylmethyl-diphenyltrisiloxanes, and mixtures thereof.

**32.** Composition according to either of Claims 30 and 31, **characterized in that** the non-volatile phenyl silicone oil may be chosen from the phenyl silicones of formula (VI) below:

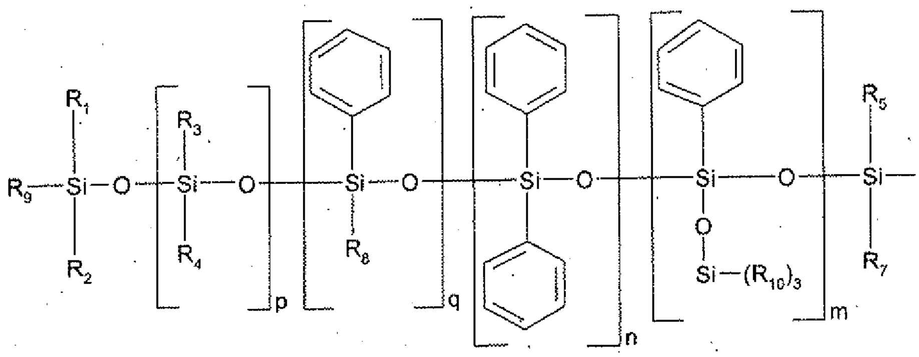

(VI)

in which

- R1 to R10, independently of each other, are saturated or unsaturated, linear, cyclic or branched C1-C30 hydrocarbon-based radicals,
- m, n, p and q are, independently of each other, integers between 0 and 900, with the proviso that the sum "m+n+q" is other than 0.

**33.** Composition according to the preceding claim, **characterized in that:**

- the sum "m+n+q" is between 1 and 100,
- the sum "m+n+p+q" is between 1 and 900 and better still between 1 and 800,
- q is equal to 0.

**34.** Composition according to Claim 32 or 33, **characterized in that** the phenyl silicone oil of formula (VI) has a viscosity at 25°C of between 5 and 1500 $mm^2/s$ (i.e. 5 to 1500 cSt) and preferably between 5 and 1000 $mm^2/s$ (i.e. 5 to 1000 cSt).

**35.** Composition according to Claim 30 or 31, **characterized in that** the non-volatile phenyl silicone oil is chosen from the phenyl silicones of formula (VII) below:

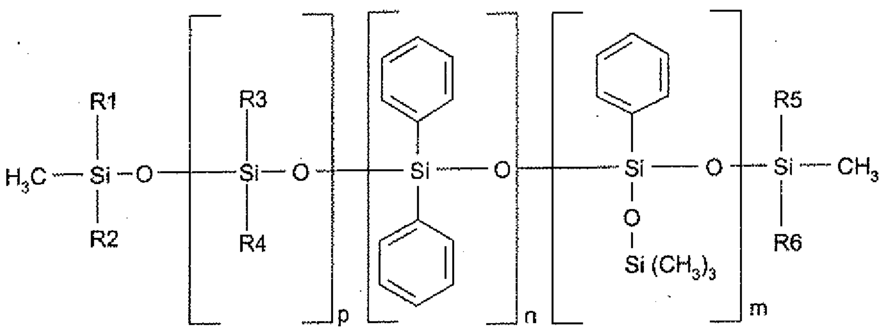

(VII)

in which:

- R1 to R6, independently of each other, are saturated or unsaturated, linear, cyclic or branched C1-C30 hydrocarbon-based radicals,
- m, n and p are, independently of each other, integers between 0 and 100, with the proviso that the sum "n+m" is between 1 and 100.

**36.** Composition according to the preceding claim, **characterized in that** R1 to R6, independently of each other, represent a linear or branched, saturated C1-C30 and especially C1-C12 hydrocarbon-based radical, and in particular a methyl, ethyl, propyl or butyl radical.

**37.** Composition according to Claim 35 or 36, **characterized in that** R1 to R6 are identical and are a methyl radical.

**38.** Composition according to any one of Claims 35 to 37, **characterized in that** m = 1 or 2 or 3, and/or n = 0 and/or p = 0 or 1.

**39.** Composition according to the preceding claim, **characterized in that** the phenyl silicone oil has a weight-average molecular weight of between 500 and 10 000.

**40.** Composition according to any one of the preceding claims, **characterized in that** the non-volatile silicone oil is present in a content ranging from 30% to 95% by weight, preferably ranging from 40% to 85% by weight and preferentially ranging from 50% to 80% by weight, relative to the total weight of the non-volatile liquid fatty phase.

**41.** Composition according to any one of the preceding claims, **characterized in that** the non-volatile silicone oil is present in a content ranging from 0.1% to 70% by weight, preferably ranging from 1% to 50% by weight and preferentially ranging from 1% to 30% by weight, relative to the total weight of the composition.

**42.** Composition according to any one of the preceding claims, **characterized in that** it comprises a non-silicone non-volatile oil.

**43.** Composition according to the preceding claim, **characterized in that** the non-silicone non-volatile oil is chosen from liquid paraffin, squalane, hydrogenated polyisobutylene, perhydrosqualene, mink oil, turtle oil, soybean oil, sweet almond oil, beauty-leaf oil, palm oil, grape seed oil, sesame seed oil, maize oil, arara oil, rapeseed oil, sunflower oil, cotton seed oil, apricot oil, castor oil, avocado oil, jojoba oil, olive oil or cereal germ oil; isopropyl myristate, isopropyl palmitate, butyl stearate, hexyl laurate, diisopropyl adipate, isononyl isononanoate, 2-ethylhexyl palmitate, 2-hexyldecyl laurate, 2-octyldecyl palmitate, 2-octyldodecyl myristate or lactate, bis(2-ethylhexyl) succinate, diisostearyl malate, glyceryl or diglyceryl triisostearate, myristic acid, palmitic acid, stearic acid, behenic acid, oleic acid, linoleic acid, linolenic acid or isostearic acid; $C_{16}$-$C_{22}$ fatty alcohols; and mixtures thereof.

**44.** Composition according to Claim 42 or 43, **characterized in that** the non-silicone non-volatile oil is present in a content ranging from 0.1% to 70% by weight, preferably ranging from 0.5% to 60% by weight and preferentially ranging from 1% to 50% by weight, relative to the total weight of the non-volatile liquid fatty phase.

**45.** Composition according to any one of Claims 42 to 44, **characterized in that** the non-silicone non-volatile oil is present in a content ranging from 0.1% to by weight, preferably ranging from 0.5% to 30% by weight and preferentially ranging from 1% to 20% by weight, relative to the total weight of the composition.

**46.** Composition according to any one of the preceding claims, **characterized in that** it comprises a volatile oil.

**47.** Composition according to any one of the preceding claims, **characterized in that** it comprises a volatile oil chosen from octamethylcyclotetrasiloxane, decamethylcyclopentasiloxane, dodecamethylcyclohexasiloxane, heptamethylhexyltrisiloxane, heptamethyloctyltrisiloxane, octamethyltrisiloxane, decamethyltetrasiloxane, isododecane, isodecane and isohexadecane.

**48.** Composition according to Claim 46 or 47, **characterized in that** the volatile oil is present in a content ranging from 0.1% to 90% by weight, preferably ranging from 1% to 70% by weight and preferentially ranging from 5% to 50% by weight, relative to the total weight of the composition.

**49.** Composition according to any one of the preceding claims, **characterized in that** it comprises at least one fatty substance that is solid at room temperature, chosen from waxes, pasty fatty substances and gums, and mixtures thereof.

**50.** Composition according to any one of the preceding claims, **characterized in that** it contains from 0.1% to 50% by weight and preferably from 1% to 30% by weight of waxes, relative to the total weight of the composition.

**51.** Composition according to any one of the preceding claims, **characterized in that** it comprises a dyestuff.

**52.** Composition according to any one of the preceding claims, **characterized in that** it comprises a cosmetic ingredient chosen from additional film-forming polymers, vitamins, thickeners, trace elements, softeners, sequestrants, fragrances, acidifying or basifying agents, preserving agents, sunscreens, surfactants, antioxidants, hair-loss counteractants, antidandruff agents and propellants, or mixtures thereof.

**53.** Cosmetic composition according to any one of the preceding claims, **characterized in that** it is in the form of a suspension, a dispersion, a solution, gel or an emulsion, especially an oil-in-water (O/W), water-in-oil (W/O) or multiple (W/O/W or polyol/O/W or O/W/O) emulsion, or in the form of a cream, a paste, a mousse, a vesicular dispersion especially of ionic or nonionic lipids, a two-phase or multi-phase lotion, a spray or a stick.

**54.** Cosmetic composition according to any one of the preceding claims, **characterized in that** it is in anhydrous form.

**55.** Cosmetic composition according to any one of the preceding claims, **characterized in that** it is a composition for making up or caring for keratin materials.

**56.** Composition according to any one of the preceding claims, **characterized in that** the composition is a lip makeup product.

**57.** Cosmetic assembly comprising:

a) a container delimiting at least one compartment, the said container being closed by a closing member; and
b) a composition placed inside the said compartment, the composition being in accordance with any one of the preceding claims.

**58.** Cosmetic assembly according to Claim 57, **characterized in that** the container is at least partially formed from at least one thermoplastic material.

**59.** Cosmetic assembly according to Claim 57, **characterized in that** the container is at least partially formed from at least one non-thermoplastic material, especially glass or metal.

**60.** Assembly according to any one of Claims 57 to 59, **characterized in that,** in the closed position of the container, the closing member is screwed onto the container.

**61.** Assembly according to any one of Claims 57 to 59, **characterized in that,** in the closed position of the container, the closing member is coupled to the container other than by screwing, especially by click-fastening, bonding or welding.

**62.** Assembly according to any one of Claims 57 to 61, **characterized in that** the composition is substantially at atmospheric pressure inside the compartment.

**63.** Assembly according to any one of Claims 57 to 61, **characterized in that** the composition is pressurized inside the container.

**64.** Cosmetic process for making up or caring for keratin materials, comprising the application to the keratin materials of a cosmetic composition of the cosmetic composition according to one of Claims 1 to 63.

**65.** Use of a composition according to one of Claims 1 to 63, to obtain a deposit on keratin materials, in particular on the skin or the lips, which has transfer-resistance properties and which is comfortable over time.

**Patentansprüche**

**1.** Kosmetische Zusammensetzung, die ein Sequenzpolymer und ein kosmetisch akzeptables, flüssiges organisches

Medium enthält, das eine nichtflüchtige flüssige Fettphase umfasst, wobei die nichtflüchtige flüssige Fettphase mindestens 30 Gew.-% nichtflüchtiges Siliconöl, bezogen auf das Gesamtgewicht der nichtflüchtigen flüssigen Fettphase, enthält,

wobei die Zusammensetzung **dadurch gekennzeichnet ist, dass** das Sequenzpolymer ein filmbildendes lineares ethylenisches Polymer ist, das mindestens eine erste Sequenz, die eine Glasübergangstemperatur von 40 °C oder darüber aufweist und die ganz oder teilweise von einem oder mehreren Monomeren abgeleitet ist, deren Homopolymer eine Glasübergangstemperatur von 40 °C oder darüber aufweist, und mindestens eine zweite Sequenz umfasst, die eine Glasübergangstemperatur von 20 °C oder darunter aufweist und die ganz oder teilweise von einem oder mehreren Monomeren abgeleitet ist, deren Homopolymer eine Glasübergangstemperatur von 20 °C oder darunter aufweist,

wobei die Monomere, deren Homopolymer eine Glasübergangstemperatur von 40 °C oder darüber aufweist, unter den folgenden Monomeren ausgewählt sind:

- Isobornylmethacrylaten,
- Isobornylacrylaten,
- und deren Gemischen,

und die Monomere, deren Homopolymer eine Glasübergangstemperatur von 20 °C oder darunter aufweist, unter den Acrylaten der Formel $CH_2$=$CHCOOR_3$ ausgewählt sind, wobei

$R_3$ eine geradkettige oder verzweigte, unsubstituierte $C_{1-12}$-Alkylgruppe mit Ausnahme der Gruppe t-Butyl bedeutet, in deren Kette gegebenenfalls ein oder mehrere Heteroatome eingebaut sind, die unter O, N und S ausgewählt sind.

2. Zusammensetzung nach Anspruch 1, **dadurch gekennzeichnet, dass** das Sequenzpolymer kein Styrol enthält.

3. Zusammensetzung nach Anspruch 1 oder 2, **dadurch gekennzeichnet, dass** das Sequenzpolymer nicht elastomer ist.

4. Zusammensetzung nach dem vorhergehenden Anspruch, **dadurch gekennzeichnet, dass** die erste Sequenz und die zweite Sequenz nicht miteinander kompatibel sind.

5. Zusammensetzung nach einem der vorhergehenden Ansprüche, **dadurch gekennzeichnet, dass** das Sequenzpolymer einen Polydispersitätsindex größer 2 aufweist.

6. Zusammensetzung nach dem vorhergehenden Anspruch, **dadurch gekennzeichnet, dass** der Anteil der ersten Sequenz im Bereich von 20 bis 90 Gew.-% des Polymers, besser im Bereich von 30 bis 80 Gew.-% und noch besser im Bereich von 50 bis 70 Gew.-% liegt.

7. Zusammensetzung nach dem vorhergehenden Anspruch, **dadurch gekennzeichnet, dass** der Anteil der zweiten Sequenz mit einer Tg von höchstens 20 °C im Bereich von 5 bis 75 Gew.-% des Polymers, besser im Bereich von 15 bis 50 Gew.-% und noch besser im Bereich von 25 bis 45 Gew.-% liegt.

8. Zusammensetzung nach einem der vorhergehenden Ansprüche, **dadurch gekennzeichnet, dass** die Sequenz, die eine Tg von 40 °C oder darüber aufweist, ganz oder teilweise von einem oder mehreren Monomeren abgeleitet ist, deren Homopolymer eine Glasübergangstemperatur im Bereich von 40 bis 150 °C, vorzugsweise von 50 °C oder darüber, insbesondere im Bereich von 50 bis 120 °C und bevorzugt von 60 °C oder darüber, insbesondere im Bereich von 60 bis 120 °C aufweist.

9. Zusammensetzung nach dem vorhergehenden Anspruch, **dadurch gekennzeichnet, dass** die Sequenz, die eine Tg von 40 °C oder darüber aufweist, ein Copolymer ist, das von Monomeren abgeleitet ist, deren Homopolymer eine Glasübergangstemperatur von 40 °C oder darüber aufweist.

10. Zusammensetzung nach einem der vorhergehenden Ansprüche, **dadurch gekennzeichnet, dass** die Sequenz, die eine Tg von 40 °C oder darüber aufweist, ein Homopolymer ist.

11. Zusammensetzung nach einem der vorhergehenden Ansprüche, **dadurch gekennzeichnet, dass** die Sequenz, die eine Tg von 20 °C oder darunter aufweist, ganz oder teilweise von einem oder mehreren Monomeren abgeleitet ist, deren Homopolymer eine Glasübergangstemperatur im Bereich von -100 bis 20 °C, vorzugsweise von 15 °C oder darunter, insbesondere im Bereich von -80 bis 15 °C und bevorzugt von 10 °C oder darunter, insbesondere

im Bereich von -50 bis 0 °C aufweist.

**12.** Zusammensetzung nach dem vorhergehenden Anspruch, **dadurch gekennzeichnet, dass** die Monomere, deren Homopolymer eine Glasübergangstemperatur von 20 °C oder darunter aufweist, unter den Alkylacrylaten ausgewählt ist, deren Alkylkette 1 bis 10 Kohlenstoffatome aufweist, wobei die Gruppe t-Butyl ausgenommen ist.

**13.** Zusammensetzung nach einem der vorhergehenden Ansprüche, **dadurch gekennzeichnet, dass** die Sequenz, die eine Tg von 20 °C oder darunter aufweist, ein Homopolymer ist.

**14.** Zusammensetzung nach einem der vorhergehenden Ansprüche, **dadurch gekennzeichnet, dass** die erste Sequenz und/oder die zweite Sequenz mindestens ein ergänzendes Monomer enthalten.

**15.** Zusammensetzung nach dem vorhergehenden Anspruch, **dadurch gekennzeichnet, dass** das ergänzende Monomer unter den hydrophilen Monomeren, Monomeren mit ethylenisch ungesättigter Bindung, die ein oder mehrere Siliciumatome enthalten, und deren Gemischen ausgewählt ist.

**16.** Zusammensetzung nach Anspruch 14 oder 15, **dadurch gekennzeichnet, dass** das ergänzende Monomer ausgewählt ist unter:

Monomeren mit einer oder mehreren ethylenisch ungesättigten Bindungen, die mindestens eine Carbonsäurefunktion oder Sulfonsäurefunktion enthalten,
Methacrylaten der Formel $CH_2=C(CH_3)-COOR_6$, wobei $R_6$ eine geradkettige oder verzweigte $C_{1-4}$-Alkylgruppe bedeutet, wobei die Alkylgruppe mit einem oder mehreren Substituenten substituiert ist, die unter der Hydroxygruppe und den Halogenatomen ausgewählt ist;
Methacrylaten der Formel $CH_2=C(CH_3)-COOR_9$, wobei $R_9$ eine geradkettige oder verzweigte $C_{6-12}$-Alkylgruppe bedeutet, in deren Kette gegebenenfalls ein oder mehrere Heteroatome eingebaut sind, die unter O, N und S ausgewählt sind, wobei die Alkylgruppe mit einem oder mehreren Substituenten substituiert ist, die unter der Hydroxygruppe und den Halogenatomen ausgewählt ist;
Acrylaten der Formel $CH_2=CH-COOR_{10}$, wobei $R_{10}$ eine geradkettige oder verzweigte $C_{1-12}$-Alkylgruppe bedeutet, die mit einem oder mehreren Substituenten substituiert ist, die unter der Hydroxygruppe und den Halogenatomen ausgewählt ist, oder $R_{10}$ eine Alkyl($C_{1-12}$)-O-POE (Polyoxyethylen) mit 5 bis 30 Oxyethylen-Wiederholungseinheiten bedeutet, oder
$R_{10}$ eine Polyoxyethylengruppe mit 5 bis 30 Ethylenoxideinheiten ist,
Monomeren mit einer oder mehreren ethylenisch ungesättigten Bindungen, die mindestens eine tertiäre Aminofunktion enthalten,

und deren Gemischen.

**17.** Zusammensetzung nach einem der Ansprüche 14 bis 16, **dadurch gekennzeichnet, dass** das oder die ergänzenden Monomere unter Acrylsäure, Methacrylsäure, Trifluorethylmethacrylat und deren Gemischen ausgewählt ist.

**18.** Zusammensetzung nach einem der Ansprüche 14 bis 17, **dadurch gekennzeichnet, dass** das oder die ergänzenden Monomere 1 bis 30 Gew.-% des Gesamtgewicht der ersten und/oder zweiten Sequenz ausmachen.

**19.** Zusammensetzung nach einem der vorhergehenden Ansprüche, **dadurch gekennzeichnet, dass** die erste und die zweite Sequenz jede mindestens ein Monomer enthalten, das unter den Estern von (Meth)acrylsäure ausgewählt ist, und gegebenenfalls mindestens ein Monomer, das unter (Meth)acrylsäure ausgewählt ist, und deren Gemische.

**20.** Zusammensetzung nach einem der vorhergehenden Ansprüche, **dadurch gekennzeichnet, dass** die erste und die zweite Sequenz jede insgesamt von mindestens einem Monomer, das unter den Estern von (Meth)acrylsäure ausgewählt ist, und gegebenenfalls mindestens einem Monomer, das unter (Meth)acrylsäure ausgewählt ist, und deren Gemischen abgeleitet ist.

**21.** Zusammensetzung nach einem der vorhergehenden Ansprüche, **dadurch gekennzeichnet, dass** das Sequenzpolymer kein Styrolmonomer enthält.

**22.** Zusammensetzung nach einem der vorhergehenden Ansprüche, **dadurch gekennzeichnet, dass** die erste Sequenz und die zweite Sequenz so gewählt sind, dass der Unterschied der Glasübergangstemperaturen (Tg) der

ersten und der zweiten Sequenz größer als 10 °C, vorzugsweise größer als 20 °C, noch bevorzugter größer als 30 °C und besonders bevorzugt größer als 40 °C ist.

**23.** Zusammensetzung nach einem der vorhergehenden Ansprüche, **dadurch gekennzeichnet, dass** die Zwischensequenz eine Glasübergangstemperatur aufweist, die zwischen den Glasübergangstemperaturen der ersten und der zweiten Sequenz liegt.

**24.** Zusammensetzung nach einem der vorhergehenden Ansprüche, **dadurch gekennzeichnet, dass** das Sequenzpolymer einen Polydispersitätsindex von mindestens 2,5, vorzugsweise mindestens 2,8 und bevorzugt im Bereich von 2,8 bis 6 aufweist.

**25.** Zusammensetzung nach einem der vorhergehenden Ansprüche, **dadurch gekennzeichnet, dass** das Sequenzpolymer eine gewichtsmittlere Molmasse (Mw) von 300000 oder darunter, vorzugsweise im Bereich von 35000 bis 200000 und noch besser im Bereich von 45000 bis 150000 aufweist.

**26.** Zusammensetzung nach einem der vorhergehenden Ansprüche, **dadurch gekennzeichnet, dass** das Sequenzpolymer eine zahlenmittlere Molmasse (Mn) von 70000 oder darunter, vorzugsweise im Bereich von 10000 bis 60000 und noch besser im Bereich von 12000 bis 50000 aufweist.

**27.** Zusammensetzung nach einem der vorhergehenden Ansprüche, **dadurch gekennzeichnet, dass** das Sequenzpolymer in einer Menge der wirksamen Substanz von mindestens 1 Gew.-% in Wasser oder einem Gemisch von Wasser und geradkettigen oder verzweigten niederen Monoalkoholen mit 2 bis 5 Kohlenstoffatomen bei Raumtemperatur (25 °C) ohne pH-Änderung unlöslich ist.

**28.** Zusammensetzung nach einem der vorhergehenden Ansprüche, **dadurch gekennzeichnet, dass** das Sequenzpolymer in einer Menge von 0,1 bis 90 Gew.-%, bezogen auf das Gesamtgewicht der Zusammensetzung, vorzugsweise 0,5 bis 50 Gew.-% und noch bevorzugter 0,5 bis 30 Gew.-% enthalten ist.

**29.** Zusammensetzung nach einem der vorhergehenden Ansprüche, **dadurch gekennzeichnet, dass** das nichtflüchtige Siliconöl unter den nichtflüchtigen Polydimethylsiloxanen (PDMS); Polydimethylsiloxanen, die als Seitenkette oder am Ende der Siliconkette Alkyl-, Alkoxy- oder Phenylgruppen enthalten, die 2 bis 24 Kohlenstoffatome aufweisen; phenylierten Siliconen; Polysiloxanen, die mit Fettsäuren (insbesondere $C_{8-20}$), Fettalkoholen (insbesondere $C_{8-20}$) oder Polyoxyalkylenen (insbesondere Polyoxyethylen und/oder Polyoxypropylen) modifiziert sind; aminierten Siliconen; Siliconen mit Hydroxygruppen; fluorierten Siliconen, die als Seitenkette oder am Ende der Siliconkette eine fluorierte Gruppe enthalten, die 1 bis 12 Kohlenstoffatome aufweist und bei der die Wasserstoffatome ganz oder teilweise durch Fluoratome ersetzt sind; und deren Gemischen ausgewählt ist.

**30.** Zusammensetzung nach einem der vorhergehenden Ansprüche, **dadurch gekennzeichnet, dass** das nichtflüchtige Siliconöl unter den nichtflüchtigen phenylierten Siliconölen ausgewählt ist.

**31.** Zusammensetzung nach einem der vorhergehenden Ansprüche, **dadurch gekennzeichnet, dass** das nichtflüchtige phenylierte Siliconöl unter den Phenyltrimethiconen, Phenyldimethiconen, Phenyltrimethylsiloxydiphenylsiloxanen, Diphenyldimethiconen, Diphenylmethyldiphenyltrisiloxanen und deren Gemischen ausgewählt ist.

**32.** Zusammensetzung nach einem der Ansprüche 30 oder 31, **dadurch gekennzeichnet, dass** das nichtflüchtige phenylierte Siliconöl unter den phenylierten Siliconölen der folgenden Formel (VI) ausgewählt ist:

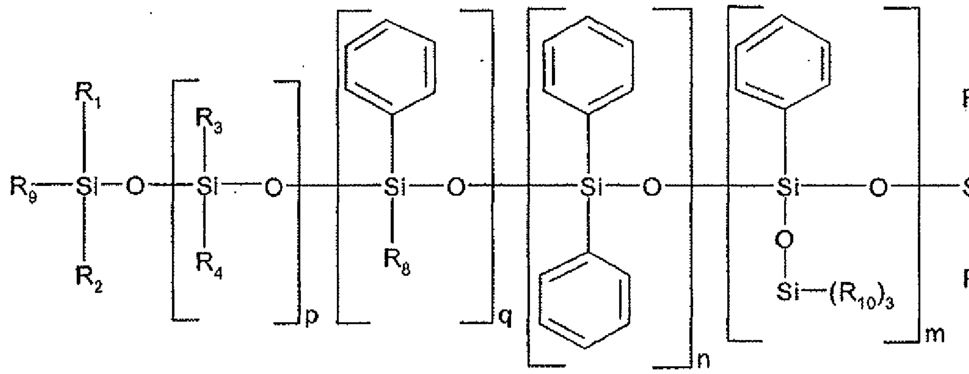

(VI)

worin bedeuten:

- R1 bis R10 unabhängig voneinander gesättigte oder ungesättigte, lineare, cyclische oder verzweigte Kohlenwasserstoffgruppen mit 1 bis 30 Kohlenstoffatomen,
- m, n, p und q unabhängig voneinander Null oder ganze Zahlen im Bereich von 1 bis 900, mit der Maßgabe, dass 'm+n+q' von Null verschieden ist.

**33.** Zusammensetzung nach dem vorhergehenden Anspruch, **dadurch gekennzeichnet, dass:**

- die Summe 'm+n+q' im Bereich von 1 bis 100 liegt,
- die Summe 'm+n+p+q' im Bereich von 1 bis 900 und besser im Bereich von 1 bis 800 liegt,
- q 0 ist.

**34.** Zusammensetzung nach Anspruch 32 oder 33, **dadurch gekennzeichnet, dass** das nichtflüchtige phenylierte Siliconöl der Formel (VI) bei 25 °C eine Viskosität im Bereich von 5 bis 1500 $mm^2/s$ (d.h. 5 bis 1500 cSt) und vorzugsweise im Bereich von 5 bis 1000 $mm^2/s$ (d.h. 5 bis 1000 cSt) aufweist.

**35.** Zusammensetzung nach einem der Ansprüche 30 oder 31, **dadurch gekennzeichnet, dass** das nichtflüchtige phenylierte Siliconöl unter den phenylierten Siliconölen der folgenden Formel (VII) ausgewählt ist:

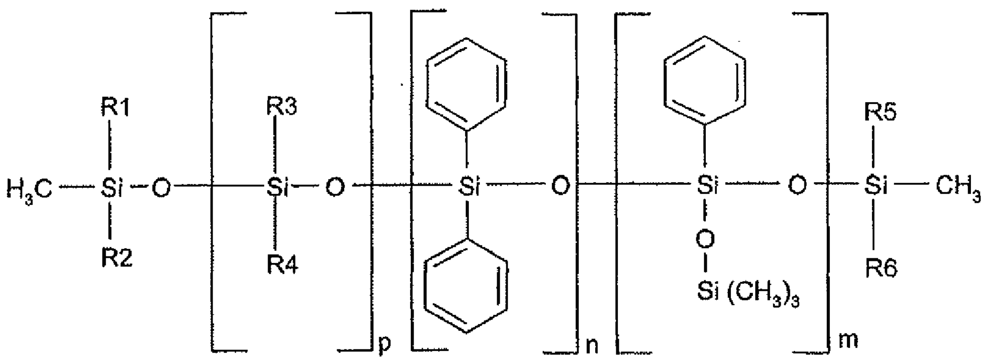

(VII)

worin bedeuten:

- R1 bis R6 unabhängig voneinander gesättigte oder ungesättigte, lineare, cyclische oder verzweigte Kohlenwasserstoffgruppen mit 1 bis 30 Kohlenstoffatomen,
- m, n und p unabhängig voneinander Null oder ganze Zahlen im Bereich von 1 bis 100, mit der Maßgabe, dass

'n+m' im Bereich von 1 bis 100 liegt.

**36.** Zusammensetzung nach dem vorhergehenden Anspruch, **dadurch gekennzeichnet, dass** die Gruppen R1 bis R6 unabhängig voneinander eine gesättigte, lineare oder verzweigte Kohlenwasserstoffgruppe mit 1 bis 30 Kohlenstoffatomen und insbesondere 1 bis 12 Kohlenstoffatomen und besonders Methyl, Ethyl, Propyl oder Butyl bedeuten.

**37.** Zusammensetzung nach Anspruch 35 oder 36, **dadurch gekennzeichnet, dass** die Gruppen R 1 bis R6 identisch sind und Methyl bedeuten.

**38.** Zusammensetzung nach einem der Ansprüche 35 bis 37, **dadurch gekennzeichnet, dass** m = 1 oder 2 oder 3 und/oder n = 0 und/oder p = 0 oder 1.

**39.** Zusammensetzung nach dem vorhergehenden Anspruch, **dadurch gekennzeichnet, dass** das phenylierte Siliconöl ein Molekulargewicht im Bereich von 500 bis 10 000 aufweist.

**40.** Zusammensetzung nach einem der vorhergehenden Ansprüche, **dadurch gekennzeichnet, dass** das nichtflüchtige Siliconöl in einem Mengenanteil von 30 bis 95 Gew.-%, bezogen auf das Gesamtgewicht der nichtflüchtigen flüssigen Fettphase, vorzugsweise im Bereich von 40 bis 85 Gew.-% und noch bevorzugter im Bereich von 50 bis 80 Gew.-% enthalten ist.

**41.** Zusammensetzung nach einem der vorhergehenden Ansprüche, **dadurch gekennzeichnet, dass** das nichtflüchtige Siliconöl in einem Mengenanteil von 0,1 bis 70 Gew.-%, bezogen auf das Gesamtgewicht der Zusammensetzung, vorzugsweise im Bereich von 1 bis 50 Gew.-% und noch bevorzugter im Bereich von 1 bis 30 Gew.-% enthalten ist.

**42.** Zusammensetzung nach einem der vorhergehenden Ansprüche, **dadurch gekennzeichnet, dass** sie ein nichtflüchtiges nichtsiliconiertes Öl enthält.

**43.** Zusammensetzung nach dem vorhergehenden Anspruch, **dadurch gekennzeichnet, dass** das nichtflüchtige nichtsiliconierte Öl ausgewählt ist unter: Paraffinöl, Squalan, hydriertem Polyisobutylen, Perhydrosqualen, Nerzöl, Schildkrötenöl, Sojaöl, Süßmandelöl, Calophyllumöl, Palmöl, Traubenkernöl, Sesamöl, Maisöl, Araraöl, Rapsöl, Sonnenblumenöl, Baumwollsamenöl, Aprikosenkernöl, Ricinusöl, Avocadoöl, Jojobaöl, Olivenöl oder Getreidekeimölen, Isopropylmyristat, Isopropylpalmitat, Butylstearat, Hexyllaurat, Diisopropyladipat, Isononylisononanoat, 2-Ethylhexylpalmitat, 2-Hexyldecyllaurat, 2-Octyldecylpalmitat, 2-Octyldodecylmyristat, 2-Octyldodecyllactat, Di(2-Ethylhexyl)-succinat, Diisostearlymalat, Glyceryltriisostearat oder Diglyceryltriisostearat, Myristinsäure, Palmitinsäure, Stearinsäure, Behensäure, Ölsäure, Linolsäure, Linolensäure oder Isostearinsäure; Fettalkoholen mit 16 bis 22 Kohlenstoffatomen; und deren Gemischen.

**44.** Zusammensetzung nach Anspruch 42 oder 43, **dadurch gekennzeichnet, dass** das nichtflüchtige nichtsiliconierte Öl in einem Mengenanteil von 0,1 bis 70 Gew.-%, bezogen auf das Gesamtgewicht der nichtflüchtigen flüssigen Fettphase, vorzugsweise im Bereich von 0,5 bis 60 Gew.-% und noch bevorzugter im Bereich von 1 bis 50 Gew.-% enthalten ist.

**45.** Zusammensetzung nach einem der Ansprüche 42 bis 44, **dadurch gekennzeichnet, dass** das nichtflüchtige nichtsiliconierte Öl in einem Mengenanteil von 0,1 bis 60 Gew.-%, bezogen auf das Gesamtgewicht der Zusammensetzung, vorzugsweise im Bereich von 0,5 bis 30 Gew.-% und noch bevorzugter im Bereich von 1 bis 20 Gew.-% enthalten ist.

**46.** Zusammensetzung nach einem der vorhergehenden Ansprüche, **dadurch gekennzeichnet, dass** sie ein flüchtiges Öl enthält.

**47.** Zusammensetzung nach einem der vorhergehenden Ansprüche, **dadurch gekennzeichnet, dass** sie ein flüchtiges Öl enthält, das unter Octamethylcyclotetrasiloxan, Decamethylcyclopentasiloxan, Dodecamethylcyclohexasiloxan, Heptamethylhexyltrisiloxan, Heptamethyloctyltrisiloxan, Octamethyltrisiloxan, Decamethyltetrasiloxan, Isododecan, Isodecan und Isohexadecan ausgewählt ist.

**48.** Zusammensetzung nach Anspruch 46 oder 47, **dadurch gekennzeichnet, dass** das flüchtige Öl in einem Mengenanteil von 0,1 bis 90 Gew.-%, bezogen auf das Gesamtgewicht der Zusammensetzung, vorzugsweise im Bereich

von 1 bis 70 Gew.-% und noch bevorzugter im Bereich von 5 bis 50 Gew.-% enthalten ist.

**49.** Zusammensetzung nach einem der vorhergehenden Ansprüche, **dadurch gekennzeichnet, dass** sie mindestens eine bei Raumtemperatur feste Fettsubstanz enthält, die unter den Wachsen, pastösen Fettsubstanzen, Gummis und deren Gemischen ausgewählt ist.

**50.** Zusammensetzung nach einem der vorhergehenden Ansprüche, **dadurch gekennzeichnet, dass** sie 0,1 bis 50 Gew.-% Wachse, bezogen auf das Gesamtgewicht der Zusammensetzung, und vorzugsweise 1 bis 30 Gew.-% enthält.

**51.** Zusammensetzung nach einem der vorhergehenden Ansprüche, **dadurch gekennzeichnet, dass** sie ein Farbmittel enthält.

**52.** Zusammensetzung nach einem der vorhergehenden Ansprüche, **dadurch gekennzeichnet, dass** sie einen kosmetischen Bestandteil enthält, der unter den ergänzenden filmbildenden Polymeren, Vitaminen, Verdickungsmitteln, Spurenelementen, beruhigenden Stoffen, Maskierungsmitteln, Parfums, Alkalisierungsmitteln oder Ansäuerungsmitteln, Konservierungsmitteln, Sonnenschutzfiltern, grenzflächenaktiven Stoffen, Antioxidantien, Wirkstoffen gegen Haarausfall, Antischuppenmitteln, Treibmitteln oder deren Gemischen ausgewählt ist.

**53.** Kosmetische Zusammensetzung nach einem der vorhergehenden Ansprüche, **dadurch gekennzeichnet, dass** sie als Suspension, Dispersion, Lösung, Gel, Emulsion und insbesondere Öl-in-Wasser-Emulsion (O/W) oder Wasser-in-Öl-Emulsion (W/O) oder multiple Emulsion (W/O/W oder Polyol/O/W oder O/W/O), als Creme, Paste, Schaum, Vesikeldispersion insbesondere von ionischen oder nichtionischen Lipiden, zweiphasige oder mehrphasige Lotion, Spray, Stick vorliegt.

**54.** Kosmetische Zusammensetzung nach einem der vorhergehenden Ansprüche, **dadurch gekennzeichnet, dass** sie in wasserfreier Form vorliegt.

**55.** Zusammensetzung nach einem der vorhergehenden Ansprüche, **dadurch gekennzeichnet, dass** es sich um eine Zusammensetzung zum Schminken oder für die Pflege von Keratinsubstanzen handelt.

**56.** Zusammensetzung nach einem der vorhergehenden Ansprüche, **dadurch gekennzeichnet, dass** die Zusammensetzung ein Produkt zum Schminken der Lippen ist.

**57.** Kosmetische Einheit umfassend:

a) einen Behälter, der mindestens eine Abteilung abgrenzt, wobei der Behälter mit einer Verschlusseinrichtung verschlossen ist, und
b) eine Zusammensetzung, die sich im Inneren der Abteilung befindet, wobei die Zusammensetzung einer Zusammensetzung nach einem der vorhergehenden Ansprüche entspricht.

**58.** Kosmetische Einheit nach Anspruch 57, **dadurch gekennzeichnet, dass** der Behälter zumindest zum Teil aus mindestens einem thermoplastischen Material gebildet ist.

**59.** Kosmetische Einheit nach Anspruch 57, **dadurch gekennzeichnet, dass** der Behälter zumindest zum Teil aus mindestens einem nichtthermoplastischen Material und insbesondere aus Glas oder Metall gebildet ist.

**60.** Einheit nach einem der Ansprüche 57 bis 59, **dadurch gekennzeichnet, dass** in der geschlossenen Stellung des Behälters die Verschlusseinrichtung mit dem Behälter verschraubt ist.

**61.** Einheit nach einem der Ansprüche 57 bis 59, **dadurch gekennzeichnet, dass** in der geschlossenen Stellung des Behälters die Verschlusseinrichtung an den Behälter auf eine andere Weise als durch Verschrauben gekoppelt ist, insbesondere durch Einrasten, Kleben oder Schweißen.

**62.** Einheit nach einem der Ansprüche 57 bis 61, **dadurch gekennzeichnet, dass** die Zusammensetzung im Inneren der Abteilung in etwa unter Atmosphärendruck steht.

**63.** Einheit nach einem der Ansprüche 57 bis 61, **dadurch gekennzeichnet, dass** die Zusammensetzung im Inneren

der Abteilung unter Druck steht.

**64.** Kosmetisches Verfahrnen zum Schminken oder für die Pflege von Keratinsubstanzen, das umfasst, eine kosmetische Zusammensetzung nach einem der Ansprüche 1 bis 63 auf die Keratinsubstanzen aufzutragen.

**65.** Verwendung einer Zusammensetzung nach einem der Ansprüche 1 bis 63, um auf den Keratinsubstanzen und insbesondere der Haut oder den Lippen eine Abscheidung zu erhalten, die sich nicht überträgt und über längere Zeit angenehm ist.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- US 4887622 A
- FR 2796529
- FR 2722380
- US 5492426 A
- FR 2761959
- WO 0103538 A
- FR 2806273
- FR 2775566
- FR 2727609
- WO 03018423 A
- FR 2791042
- FR 2792618